# EUROPEAN PATENT APPLICATION

(11) **EP 4 549 558 A1**
(43) Date of publication of application: **07.05.2025**
(21) Application number: 23828679.3
(22) Date of filing: 26.06.2023
(51) Int. Cl.: C12N 7/01, A61K 35/76, A61K 48/00, A61P 31/04, C12N 15/31, C12N 15/70, C12N 15/74

(54) **BACTERIA-TARGETING CAPSID PARTICLE, TREATMENT COMPOSITION, DISINFECTANT, FOOD, METHOD FOR ELIMINATING BACTERIA, STERILIZATION METHOD, METHOD FOR PREVENTING DECAY, METHOD FOR TREATING ANIMAL, METHOD FOR INTRODUCING GENE, METHOD FOR ADDING BACTERIA FUNCTION, METHOD FOR PRODUCING BACTERIA-TARGETING CAPSID PARTICLE, AND METHOD FOR PRODUCING NUCLEIC ACID FOR BACTERIA-TARGETING CAPSID PARTICLE**

(30) Priority: 29.06.2022 JP 2022104184
(71) Applicant: Jichi Medical University, Tokyo 102-0093 (JP)
(72) Inventor: CUI, Longzhu, Shimotsuke-shi, Tochigi 329-0498 (JP); KIGA, Kotaro, Shimotsuke-shi, Tochigi 329-0498 (JP)
(74) Representative: Grünecker Patent- und Rechtsanwälte PartG mbB
(86) International application number: PCT/JP2023/023521
(87) International publication number: WO 2024/004923

(57) **Abstract**

Provided is a bacteria-targeting capsid particle that is non-proliferative and has a high bactericidal effect. A capsid protein of a bacteriophage is prepared by a capsid nucleic acid element that synthesizes the capsid, which is divided from the bacteriophage genome and does not include a packaging region, but mainly includes the virion region. A bacteria-targeting capsid particle element (Bacteria-targeting capsid particle, B-CAP) is divided from a part of the bacteriophage genome other than the capsid nucleic acid element and includes a nucleic acid injection region, a replication region necessary for nucleic acid replication, and a packaging region. Although the assembled bacteria-targeting capsid particle (B-CAP) is non-proliferative, it can carry long DNA strands that give them new biological functions, such as bactericidal effects, or the other biological functions.

## Description

### BACKGROUND

### (Technical Field)

The present disclosure especially relates to, in particular, a bacteria-targeting capsid particle, treatment composition, disinfectants, food, a bacteria elimination method, a bactericidal method, a corrosion prevention method, an animal treatment method, a gene introduction method, a bacteria function addition method, a method for producing a bacteria-targeting capsid particle, and a method for producing nucleic acid for a bacteria-targeting capsid particle.

### (Background technology)

Typically, a lot of antibacterial drugs have been developed and various bacterial infections have been treated. However, soon after the use of antibacterial drugs, drug-resistant bacteria (bacteria that are not affected by antibacterial drugs or antibiotics) began to be reported. Now, drug-resistant bacteria exist everywhere on the Earth, and drug-resistant bacteria have appeared against almost all antibacterial drugs used in clinical practice. Since resistant bacteria are appearing at a rate far faster than the development of antibacterial drugs, bacterial infections are once again becoming a global problem that threatens human health. In other words, looking at the history of antibacterial drug development and the evolution of resistant bacteria, the emergence of resistant bacteria seems inevitable, and there is a fear that there are no evolution-adaptive drugs that can respond to the emergence of new resistant bacteria. In such critical situations, countries around the world are formulating action plans to combat drug resistance and promoting the development of new prevention, diagnosis, and treatment methods for resistant bacteria.

Here, typically, bacteriophage therapy, which is an antibacterial treatment method by using a bacteriophage (hereinafter referred to as a "phage"), has been known. The bacteriophages are viruses that infect bacteria, and they proliferate through the following process: (1) attaching to the host bacteria, (2) injecting their own nucleic acid (DNA or RNA), and (3) replicating their own nucleic acid within the bacteria. After that, (4) synthesizing the capsid protein that forms the outer shell, (5) assembling daughter bacteriophage, and (6) releasing the daughter bacteriophage outside to the bacterium through lysis, or the like, are processed. The bacteriophage therapy is a treatment that uses the lysis activity of bacteriophages, which are viruses that infect bacteria, to sterilize them, and it is first attempted in 1915 with the discovery of bacteriophages, and in recent years, clinical applications have been accelerating.

However, since the bacteriophages multiply and transmit genes, there are concerns about the induction of unexpected bacterial evolution, the transmission of toxin genes, and adverse effects on the ecosystem.

Therefore, the patent document 1 discloses a bactericidal method by using a bacteriophage carrying CRISPR-Cas13 that does not multiply. In addition, attempts have also been made to develop bacteriophage therapy by using gene-deficient bacteriophages and bacteriophagemids.

### (Prior art)

### (Patent documents)

(Patent document 1) WO2014/204726

### (Non-patent documents)

(Non-patent document 1) Kiga K. et al., "Development of CRISPR-Cas13a-based antimicrobials capable of sequence-specific killing of target bacteria", Nature Communications sequence-specific killing of target bacteria", Nature Communications, 2020, vol. 11(1): 2934.
(Non-patent document 2) De Graaf, F. K., and Oudega, B., "Production and release of clostridin DF13 and and related colicins", Curr. Top. Microbiol. Immunol., 1986, vol. 125, pp. 183-205.

### SUMMARY OF THE INVENTION

### (Problems to be solved by the Invention)

However, there are limitations with typical non-proliferative bacteriophages, such as the fact that they can only kill bacteria that have been infected, and there is a demand for bacteriophages that are more effective in treating drug-resistant bacteria, or the like.

The present disclosure is made in light of this situation, and the objective is to resolve the above-mentioned issues.

### (Means for resolving the problem)

A bacteria-targeting capsid particle according to the present disclosure is a bacteria-targeting capsid particle including: a capsid protein of a bacteriophage; and a bacteria-targeting capsid particle element including a nucleic acid injection region, a replication region necessary for replication of nucleic acid, and a packaging region in genome of the bacteriophage; and wherein being non-proliferative.

The bacteria-targeting capsid particles according to the present disclosure is, wherein the bacteria-targeting capsid particle element includes an exogenous gene.

The bacteria-targeting capsid particles according to the present disclosure is, wherein the exogenous gene includes one or any combination of a bactericidal gene, a biofilm-degrading gene, an antigen-presenting gene, and a gene for introduction.

The bacteria-targeting capsid particles according to the present disclosure is, wherein producing a secretory bactericidal product that sterilizes surrounding bacteria by the bactericidal gene.

The bacteria-targeting capsid particles according to the present disclosure is, wherein the exogenous gene includes a resistance factor that suppresses the bactericidal effect on the target bacteria.

A treatment composition according to the present disclosure includes the bacteria-targeting capsid particle.

A disinfectant according to the present disclosure includes the bacteria-targeting capsid particle.

A food according to the present disclosure includes the bacteria-targeting capsid particle.

A bacteria elimination method according to the present disclosure including the step of: sterilizing target bacteria by the bacteria-targeting capsid particle.

The bacteria elimination method according to the present disclosure is, wherein the target bacteria are present in bacterial flora of a human, an animal, and/or an environment.

The bacteria elimination method according to the present disclosure is, wherein the target bacteria are present in food.

A bactericidal method according to the present disclosure including the step of: sterilizing target bacteria with the bacteria-targeting capsid particle.

A corrosion prevention method according to the present disclosure including the steps of: sterilizing target bacteria with the bacteria-targeting capsid particle; and preventing corrosion of an article.

An animal treatment method according to the present disclosure including the step of: treating an animal with the bacteria-targeting capsid particle.

A gene introduction method according to the present disclosure including the step of: introducing the exogenous gene included in the bacteria-targeting capsid particle according to any one of claims 2 to 5 into the target bacteria.

A bacteria function addition method according to the present disclosure including the steps of: introducing the exogenous gene included in the bacteria-targeting capsid particles according to any one of claims 2 to 5 into the target bacteria; and adding a function to the target bacteria.

A method for producing bacteria-targeting capsid particle according to the present disclosure including the steps of: preparing a capsid protein of a bacteriophage by a capsid nucleic acid element that synthesizes a capsid without including a packaging region and is divided from genome of a bacteriophage; packaging a bacteria-targeting capsid particle element, which includes a nucleic acid injection region, a replication region necessary for nucleic acid replication, and a packaging region and is divided from a part of the genome of the bacteriophage other than the capsid nucleic acid element, into the capsid protein.

The method for producing the bacteria-targeting capsid particles according to the present disclosure, further including the steps of: introducing the capsid nucleic acid element and the bacteria-targeting capsid particle element into a preparation bacteria for preparation; and producing the bacteria-targeting capsid particles in the preparation bacteria.

The method for producing the bacteria-targeting capsid particles according to the present disclosure is, wherein introducing the capsid nucleic acid element by a chromosome or an artificial chromosome for the preparation bacteria.

The method for producing the bacteria-targeting capsid particles according to the present disclosure is, wherein introducing the bacteria-targeting capsid particle element by the chromosome or a plasmid.

A method for producing nucleic acid for bacteria-targeting capsid particle according to the present disclosure including the steps of: preparing a capsid nucleic acid element that synthesizes a capsid without including a packaging region by dividing a bacteriophage genome; preparing a bacteria-targeting capsid particle element including a nucleic acid injection region, a replication region necessary for replication of nucleic acid, and the packaging region by dividing a part of the bacteriophage genome other than the capsid nucleic acid element; and constructing a nucleic acid for the bacteria-targeting capsid particle that is non-proliferative.

(Effect of the Invention) According to the present disclosure, by providing a bacteriophage capsid protein and a bacteria-targeting capsid particle elements that includes a nucleic acid injection region, a replication region necessary for nucleic acid replication, and a packaging region, it is possible to provide a bacteria-targeting capsid particle that is non-proliferative and effective for the treatment of drug-resistant bacteria, or the like.

### BRIEF DESCRIPTION OF THE DRAWINGS

FIG. 1 is a diagram showing a structure of a bacteria-targeting capsid particle according to the present embodiment.
FIG. 2 is a conceptual diagram showing a comparison between typical bacteriophage therapy and an antibacterial treatment method by using the bacteria-targeting capsid particle according to the present embodiment.
FIG. 3A is a conceptual diagram showing the sequence structure of T7 bacteriophage in an example according to the present embodiment.
FIG. 3B is a conceptual diagram showing the sequence structure of helper B-CAP in the example according to the present embodiment.
FIG. 4 is a conceptual diagram of the sequence structure of T7 bacteriophage and B-CAP in the example according to the present disclosure.
FIG. 5A is a schematic diagram of a result of electrophoresis confirming the construction of B-CAP by using PCR method in the example according to the present disclosure.
FIG. 5B is a photograph of the results of electrophoresis confirming the construction of B-CAP using the PCR method in the example according to the present disclosure.
FIG. 6A is a photograph of T7 bacteriophage by using an electron microscope in the example according to the present disclosure.
FIG. 6B is a photograph of B-CAP by using an electron microscope in the example according to the present disclosure.
FIG. 7 is a photograph showing the bactericidal activity of B-CAP in the example according to the present disclosure.
FIG. 8A is a B-CAP sequence structure of Tan2 bacteriophage derived from Staphylococcus aureus in the example according to the present disclosure.
FIG. 8B is a conceptual diagram of B-CAP synthesis of Tan2 bacteriophage derived from Staphylococcus aureus in the example according to the present disclosure.
FIG. 8C is a photograph of B-CAP synthesis of Tan2 bacteriophage derived from Staphylococcus aureus in the example according to the present disclosure.
FIG. 9 is a conceptual diagram of a test for loading long DNA onto B-CAP in the example according to the present disclosure.
FIG. 10 is a graph showing the number of plaques obtained in the test as shown in FIG. 9.
FIG. 11 is a photograph of the plaques obtained in the test as shown in FIG. 9.
FIG. 12A is a schematic diagram showing the confirmation of the loading of long DNA in the plaques obtained in the test as shown in FIG. 9 by using the PCR method.
FIG. 12B is a photograph of the electrophoresis results in the test as shown in FIG. 9 by using the PCR method.
FIG. 13A is a figure showing a conceptual diagram of confirming the loading of long DNA in the plaque obtained in the test in FIG. 9.
FIG. 13B is a figure showing a result of confirming the loading of long DNA in the plaque obtained in the test in FIG. 9 by sequencing.
FIG. 14 is a conceptual diagram of the construction of a bactericidal B-CAP in the example according to the present disclosure.
FIG. 15 is a schematic diagram of the sequence structure of the bactericidal B-CAP in the example according to the present disclosure.
FIG. 16 is a photograph showing the bactericidal effect of the bactericidal B-CAP in the plate in the example according to the present disclosure.
FIG. 17A is a graph showing the bactericidal effect of the bactericidal B-CAP in liquid culture in the example according to the present disclosure.
FIG. 17B is a graph showing the bactericidal effect of the bactericidal B-CAP in liquid culture in the example according to the present disclosure.
FIG. 18 is a photograph showing the bactericidal effect of the bactericidal B-CAP on drug-resistant E. coli in the example according to the present disclosure.
FIG. 19A is a conceptual diagram of the antibacterial treatment test on mice infected with bacteria by using the bactericidal B-CAP in the example according to the present disclosure.
FIG. 19B is a conceptual diagram of the antibacterial treatment test on mice infected with bacteria by using the bactericidal B-CAP in the example according to the present disclosure.
FIG. 20A is a graph showing the antibacterial therapeutic effect of the bactericidal B-CAP on mice infected with bacteria in the example according to the present disclosure.
FIG. 20B is a graph showing the antibacterial therapeutic effect of the bactericidal B-CAP on mice infected with bacteria in the example according to the present disclosure.

### DETAILED DESCRIPTION

### <Embodiment>

There are concerns that proliferative bacteriophages may induce unexpected bacterial evolution, spread toxin genes, and have a negative impact on the ecosystem.

For this reason, the inventors of the present invention have motives to create a non-proliferative bacteriophage that is effective in treating drug-resistant bacteria. After repeated experiments, the inventors of the present invention conceive of constructing a "non-proliferative bacteriophage capsid particle that can inject DNA into bacteria," which has bacteriophage genome elements necessary for DNA injection and DNA replication. The inventors of the present invention therefore generate a DNA construction (element) with a sequence that excludes the virion component genes, which include the region that synthesizes the capsid and relates to proliferation, and succeed in packaging the element into the lysogenic bacteriophage capsid, thereby completing the present invention. The element packaged within the bacteriophage capsid is injected into the cell of the target host bacteria in the same way as bacterial infection by a wild bacteriophage. Thus, the element packaged in this way is given to be named a non-proliferative bacteria-targeting capsid particle (Bacteria-targeting capsid particle, hereinafter referred to as "B-CAP").

In the following, it describes the B-CAP, treatment composition, disinfectant, food, bacteria elimination method, bactericidal method, corrosion prevention method, animal treatment method, gene introduction method, bacterial function addition method, Method for producing B-CAP, and Method for producing nucleic acid for B-CAP in concrete detail.

### (Construction of B-CAP)

Firstly, we explain a method for constructing B-CAP (a production method). At first, as a method for dividing a bacteriophage genome in the present embodiment, the bacteriophage chromosome (genome) is divided appropriately to construct a B-CAP element specialized for transduction, DNA replication, and packaging in the lysogenic bacteriophage. Specifically, the bacteriophage genome is divided to produce the B-CAP element that includes only the genes necessary for DNA injection and DNA replication. This B-CAP element excludes the virion (viral) structural genes, which are related to proliferation and include a region that synthesizes the capsid, and includes the nucleic acid injection region, the replication region necessary for nucleic acid replication, and the packaging region.

The B-CAP element may be constructed by using a chromosome or a plasmid. This plasmid may have a higher copy number than the artificial chromosome.

On the other hand, from the genome of the bacteriophage, the construction of the capsid nucleic acid element (hereinafter referred to as "capsid"), which is a DNA element for constructing the capsid that synthesizes the bacteriophage capsid, that is, the region other than the B-CAP element, is also carried out. The capsid nucleic acid element includes the virion but does not contain the packaging region, and it is specialized for synthesizing the capsid.

In the present embodiment, the capsid nucleic acid element may be constructed by using a chromosomal or bacterial artificial chromosome (BAC) for the preparation bacteria in order to stably maintain it within the preparation bacteria.

In this way, by constructing the B-CAP element and the capsid nucleic acid element, it is possible to produce non-proliferative B-CAP nucleic acid.

The B-CAP element and capsid nucleic acid element can be introduced into the preparation bacteria for preparation, and it is possible to produce B-CAP in the preparation bacteria. Specifically, the B-CAP element and capsid nucleic acid element can be introduced into the bacteria used to prepare B-CAP by electroporation, liposome, injection, or other gene introduction methods.

The preparation bacteria can be, for example, common bacteria such as Escherichia coli, Bacillus subtilis, and the like. It is also possible to use bacteria of the same species or a closely related species of a target bacteria, which B-CAP infects and injects DNA into, as the preparation bacteria.

Then, in the method for producing B-CAP in the present embodiment, the B-CAP element is grown within the preparation bacteria. In the same preparation bacteria, the capsid protein is also produced by the capsid nucleic acid element, and the grown B-CAP element is packaged in this capsid protein. This results in the formation of the non-proliferative B-CAP particle. The B-CAP particle can be obtained in large quantities by methods commonly used by those skilled in the art, where the preparation bacteria is lysed and/or released from the cells and purified by ultracentrifugation, or the like.

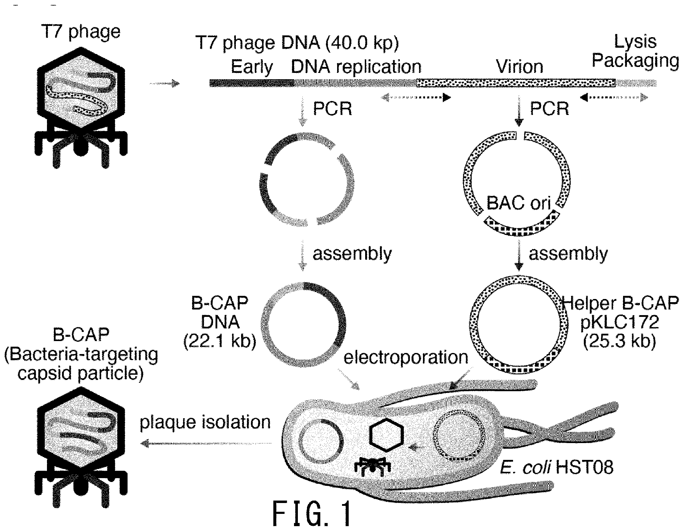

FIG. 1 shows an example of constructing B-CAP by using T7 bacteriophage, which mainly infects E. coli.

In the present embodiment, as shown in the example described later, the genome of T7 bacteriophage, which is a model for proliferating bacteriophage, is divided into a B-CAP element and the region of the helper B-CAP, which is a capsid nucleic acid element, and assembled them. Then, DNA of the B-CAP element and pKLC172 are electroporated into E. coli strains HST08, MC1061, and MC1061R, respectively, and thus packaged into the capsid of T7 bacteriophage. This enables the generation of B-CAP, which can introduce DNA into the target bacteria.

The B-CAP produced in this way is non-proliferative. That means, it can only proliferate within the preparation bacteria having the capsid nucleic acid element, which is the other half that has been divided, and the preparation bacteria becomes the "factory" for B-CAP synthesis.

On the other hand, the produced B-CAP has the same infection manner as a wild-type bacteriophage, although it is non-proliferative, and can deliver long DNA to the target bacteria. In other words, B-CAP is capable of carrying foreign DNA and injecting B-CAP element DNA into target bacteria. In addition, the B-CAP element that has been injected can exert biological activity derived from its own DNA, including amplification within the target bacteria.

Therefore, B-CAP can be applied more efficiently than before as an antibacterial therapeutic agent for bacterial infections, disinfectant, intestinal regulator, oral component, preservative, or the like.

In the present embodiment, the B-CAP element of B-CAP may contain an exogenous gene. The exogenous gene may include any or a combination of a biofilm-degrading gene, an antigen-presenting gene, a gene for introduction, and a bactericidal gene.

Specifically, as shown in the example described later, it is possible to insert at least 18.0 kb of foreign long DNA into the B-CAP that has been generated. It is possible to include the exogenous gene within this long DNA.

Among these, the biofilm-degrading gene in the present embodiment may be, for example, a dextranase or protease that breaks down or degrades the molecular structure of the biofilm composed of bacterial polysaccharides, peptidoglycans, proteins, or the like. The gene product of this biofilm-degrading gene degrades the bacterial biofilm, making it impossible for the bacteria itself to survive, and/or making an antibacterial agent, or the like, easier to immerse.

Also, the antigen presentation gene in the present embodiment may be a gene that makes it easier for the immune system to recognize or presents an antigen that is a target for immune attack. This antigen does not necessarily have to be an antigen for the bacteria that B-CAP infects, but it may be an antigen for other pathogenic organisms, viruses, animal tumors, or the like, which exist in the lesion. In other words, because the B-CAP in the present embodiment can freely introduce DNA sequences into the capsid, it can also be used in the field of bacteriophage vaccines, where DNA from pathogenic bacteria, intestinal flora bacteria, and other bacteria can be loaded, or antigens can be presented on the surface of the bacteriophage capsid. It can also be applied to cancer treatment and the treatment of hereditary diseases.

Also, the gene for introduction according to the present embodiment may be a gene that is introduced into bacteria. The gene for introduction can use various genes to add functions to the bacteria, which B-CAP infects. For example, genes involved in the synthesis and secretion of proteins and various substances, and the like, can be included as the additional function. Also, as mentioned above, B-CAP can carry foreign long DNA, so it is possible to introduce a plurality of genes. These introduced genes can be, for example, drug genes, drug resistance genes, genes encoding proteins related to attenuation or virulence, toxin genes, genes for specific metabolites, enzymes for producing specific metabolites genes, genes for identifying bacteria, reporter genes used for transformation, sequences used for restriction enzymes or sticky ends used for genetic modification, repeat sequences, and other "genes" that indicate a genotype of broad sense.

Also, the exogenous gene of the present embodiment may cause the loss of drug resistance or the generation of drug resistance by nucleic acid acquisition and/or nucleic acid mutation.

Specifically, because resistant bacteria can be generated by the acquisition of foreign DNA or RNA (nucleic acid) and/or nucleic acid mutation, it is possible to enhance the antibacterial effect by using a exogenous gene that suppresses this nucleic acid acquisition and/or nucleic acid mutation.

Also, the bactericidal gene in the present embodiment may be one that generates a secretory bactericidal product. Specifically, it is possible to generate a secretory bactericidal product that kills bacteria surrounding itself by using the bactericidal gene in the present embodiment. In other words, by loading the bactericidal gene that generates a secretory bactericidal product onto B-CAP in the present embodiment, it is possible to kill target bacteria and the bacteria group around the target bacteria (mass sterilization).

For example, it is possible to load secretory bactericidal DNA machinery as an exogenous gene into B-CAP. This bactericidal DNA machinery can, for example, be loaded with a bacteriocin gene cluster (colicin E1 gene cluster) for producing and secreting the bacteriocin. The Bacteriocin is an antibacterial protein or a peptide produced by bacteria, secreted outside the bacteria, and exhibit antibacterial activity against related bacteria. Therefore, by incorporating a bacteriocin gene cluster into B-CAP, it is possible to sterilize target bacteria and bacteria group in the surrounding area, and a significant antibacterial effect can be obtained.

The inventors have named this type B-CAP, which is used as a bactericidal agent, the "non-proliferative mass-killing antibacterial capsid. It is hereafter referred to as "NM-AB capsid."

The NM-AB capsid in the present embodiment shows an extraordinarily strong mass killing effect and is a B-CAP that is non-proliferative but can be efficiently treated.

Specifically, as shown in the examples later, one example of the NM-AB capsid is a B-CAP (B-CAP_ColE1) that carries a bacteriocin gene cluster, and it is non-proliferative, but it exhibits strong bactericidal activity against carbapenem-resistant E. coli. It also exhibits a significant survival effect on mice infected with this bacterium.

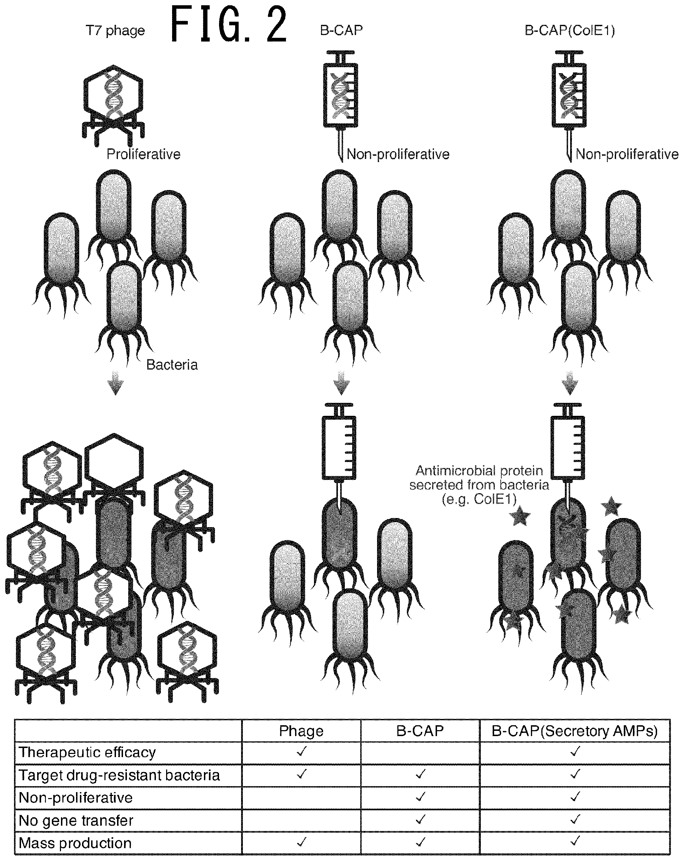

As refer to FIG. 2, a concept of the antimicrobial treatment method by using B-CAP having the NM-AB capsid is explained.

FIG. 2 shows the differences between the antimicrobial treatments provided by T7 bacteriophage, B-CAP, and B-CAP_ColE1.

T7 bacteriophage is a proliferative that multiplies within the cell bodies of the target bacteria, lysing the host in the process of proliferation. Because it proliferates, there are concerns about its negative impact on the environment and the transmission of its genes to the other bacteria.

On the other hand, B-CAP can kill target bacteria by injecting DNA derived from bacteriophages, but its bactericidal effect itself is not strong because it is not proliferative. On the other hand, B-CAP can carry long genes, and it is possible to transiently overexpress the gene within the target bacteria. For this reason, it can be used in numerous ways as a non-proliferative capsid. Furthermore, it can be used efficiently for sterilization, or the like, by incorporating the above-mentioned biofilm removal gene, or the like.

B-CAP_ColE1 is an example of the NM-AB capsid and is a B-CAP, which loads secretory antimicrobial proteins/peptides (AMPs). B-CAP_ColE1 loads the bacteriocins as AMPs. Once the NM-AB capsid infected inside the target bacteria, it amplifies, and a large amount of AMPs is synthesized in the target bacteria. The AMPs are secreted outside the bacteria and can also kill other surrounding bacteria.

Here, in the present embodiment, B-CAP_ColE1 is generated by loading Colicin E1 (ColE1), immunity protein (immunity gene), and colicin release lysis protein (secretion gene) onto a B-CAP as bacteriocin gene cluster for the B-CAP_ColE1.

Among these, the immunity protein (immune gene) is an example of a resistance factor that suppresses the sterilization of target bacteria. In this way, by including the gene for a resistance factor as an exogenous gene in B-CAP, it is possible to suppress the killing of the target bacteria until a sufficient amount of secretory bactericidal products have been produced after the NM-AB capsid has infected to the target bacteria.

In addition, the exogenous genes to be loaded onto the B-CAP in the present embodiment may be configured to be multiple. In other words, the B-CAP of the present embodiment may have a plurality of exogenous genes within a single bacteriophage. In this case, the plurality of exogenous genes may be included within the range that can be contained within the capsid. The plurality of exogenous genes may be sequentially arranged within a sequence or as multiple sites, or they may be configured to include multiple nucleotides.

For example, by incorporating the plurality of bactericidal genes that can be applied to multiple types of bacteria, a single B-CAP can be applied to multiple resistant bacteria. Furthermore, in the case of multidrug-resistant bacteria that have multiple resistance genes, it is also possible to improve the antibacterial efficiency by loading the plurality of genes that correspond to all of these. Furthermore, it is also possible to further improve the antibacterial effect by loading B-CAP with any combination of the above-mentioned bactericidal genes, biofilm-degrading genes, antigen-presenting genes, and introduction genes.

### (Treatment composition)

The B-CAP in the present embodiment can also be used in treatment compositions containing it.

In other words, by administering the B-CAP in the present embodiment for therapeutic purposes, it can be used in antibacterial treatment methods that kill bacteria. Specifically, B-CAP can be used to achieve an antibacterial treatment that kills bacteria. This makes it possible to treat infections that are difficult to treat with antibacterial drugs, including infections caused by drug-resistant bacteria.

Various compositions similar to those used in typical bacteriophage-based antibacterial treatments can be used to manufacture the treatment composition.

Also, the treatment composition according to the embodiment of the present disclosure can be made by using any carrier that is acceptable for use in a pharmaceutical formulation (that is, isotonic solutions containing physiological saline, glucose, and other auxiliary agents, such as D-sorbitol, D-mannose, D-mannitol, and sodium chloride, or the like, and appropriate solubilizing agents, such as alcohols, specifically ethanol, poly-alcohols, such as propylene glycol, polyethylene glycol, nonionic surfactants, such as polysorbate 80 (TM), HCO-50, or the like, but not limited to these), can be administered together. It may also contain appropriate excipients, or the like.

Also, the treatment composition in the present embodiment may also include a suitable pharmaceutically acceptable carrier for preparing a formulation-acceptable support. The carrier may include biocompatible materials such as silicone, collagen, gelatin, or the like. Alternatively, it may be a variety of emulsions. Furthermore, it may contain one or more pharmaceutical additives selected from diluents, fragrances, preservatives, excipients, disintegrants, lubricants, binders, emulsifiers, plasticizers, or the like.

The treatment composition according to the present embodiment may be formulated in a dosage form suitable for oral administration by using a carrier that is acceptable in the formulation and is well known in the field.

The administration route of the pharmaceutical composition according to the present disclosure is not particularly limited, and it is possible to administer it orally or parenterally. For parenteral administration, for example, it is possible to administer it intravenously, intra-arterially, subcutaneously, intradermally, intramuscularly, intraperitoneally, or to administer it directly to the bacterial flora or infected site, drip, or apply it. This method of administration may be conducted in an equivalent way to typical bacteriophage therapy.

Also, in order to use the treatment composition according to the present embodiment for the above-mentioned treatment, the administration interval and dosage may be selected and changed as appropriate according to various conditions such as the condition of the disease and the condition of the subject.

The dosage and number of administrations of the treatment composition according to the present embodiment can be selected and changed as appropriate according to the purpose of administration and various conditions such as the patient's age and weight, symptoms, severity of the disease, and the like.

The number of doses and the duration of treatment may be administered once only, once or several times a day for several weeks, and the condition of the disease may be monitored, and the dose may be administered again or repeatedly depending on the condition.

The treatment composition according to the present embodiment can also be used in combination with other compositions, or the like. Also, the present composition can be administered at the same time as the other compositions, or it can be administered at intervals, but the order of administration is not particularly important.

In addition, in the embodiments according to the present disclosure, the period during which the disease is improved or alleviated is not particularly limited, but may be a temporary improvement or alleviation, or it may be an improvement or alleviation for a certain period of time.

Also, the treatment composition of the embodiments according to the present disclosure may be used for the treatment of a living organism, a part of the living organism, or a part of the living organism that has been removed or expelled from the living organism.

The living organism is not particularly limited, and may be, for example, an animal, a plant, or a fungus. The animal may include, for example, humans, domestic animal species, and wild animals.

Therefore, the treatment composition according to the embodiment of the present disclosure can also be used for animal therapy to treat animals. In other words, B-CAP in the present the embodiment can also be used for animal therapy methods targeting various animals other than human.

Specifically, the animal treatment method in the present embodiment can be used to treat bacterial infections in animals other than humans by using a treatment composition containing B-CAP.

The animals also include a wide range of vertebrates and invertebrates. Vertebrates include fish, amphibians, reptiles, birds, and mammals. More specifically, for example, mammals may include rodents, such as mice, rats, ferrets, hamsters, guinea pigs, or rabbits, or dogs, cats, sheep, pigs, cows, horses, or non-human primates. In addition to mammals, wild animals also include fish, birds including poultry, and reptiles. They also include crustaceans including shrimp and insects, and other invertebrates such as squid.

In other words, the treatment composition according to the present embodiment can be used not only for the treatment of humans, but also for the treatment of animals and the promotion of growth in livestock.

Also, the bactericidal method according to the present embodiment performs the removal (sterilization, pasteurization) of target bacteria by B-CAP.

As such, it is possible to use the composition including B-CAP according to the present embodiment for the purpose of a disinfectant (growth inhibitor). As mentioned above, the disinfectant can also be included in an appropriate carrier, solution, or the like. In other words, the composition including B-CAP in the present embodiment may be provided as a sterilizing agent and included in various antibacterial products, such as detergents, hand soap, mouthwash, masks and napkins, air purifier filters, or the like.

Also, the composition including B-CAP in the present embodiment can also be used for the purpose of removing bacterial peptidoglycans, biofilms, or the like. In such case, as mentioned above, it is suitable to load a biofilm-degrading gene as an exogenous gene of B-CAP.

By applying, spraying, or the like, this disinfectant, it is possible to remove bacteria from lesions, bacterial colonies, or the environment and to inhibit their growth. In other words, it is possible to reduce the number of bacteria present in the bacterial colonies of humans, animals, and/or the environment.

In addition, the bacteria elimination method according to the present embodiment can also be used to remove bacteria present inside or outside of food. Specifically, it is also possible to provide food that includes B-CAP. That means, B-CAP according to the present embodiment can be used to remove bacteria from food. This is because B-CAP according to the present embodiment does not infect humans even if it is ingested. It is also possible to provide food that has been treated in this way to remove bacteria. This makes it possible to provide, for example, safe food that has been reliably sterilized of food poisoning bacteria that produce toxins. Here, B-CAP in the present embodiment can be used by including it in any food that has been cultivated, farmed, or harvested, including various vegetables, meat, seafood, processed foods, and dairy products.

Furthermore, B-CAP in the present embodiment can also be used for the purpose of corrosion prevention method that sterilizes target bacteria and prevents the corrosion of food and non-food items.

As configured in this way, the following effects can be obtained.

Typically, antibiotics have played the most significant role in the treatment of bacterial infections. However, the emergence of drug-resistant bacteria that are resistant to existing antibiotics and their rapid spread are rendering existing antibiotics ineffective. Since drug-resistant bacteria are spreading on a global scale, there is a need to develop new antibiotics. However, the emergence of drug-resistant bacteria is outpacing the development of new antibiotics, and the development of new antibiotics has stalled, making it increasingly difficult to solve the problem of drug-resistant bacteria. As a result, bacterial infections have once again become a global problem that threatens human health.

Further, there is a movement to develop bacteriophage therapy, as the bactericidal effect of bacteriophages on drug-resistant bacteria has been recognized. The bacteriophages used in bacteriophage therapy multiply within the bacteria present in the infection site, so they exhibit strong bactericidal activity. However, due to the biological properties of the bacteriophages themselves, which are capable of reproducing, there are concerns about their clinical use, and their practical application has not progressed. In other words, when the reproductive properties of bacteriophages are used in antibacterial treatment, it may have the bactericidal effect, but there are concerns about adverse effects on the ecosystem and unintended side effects. There are also concerns about the spread of drug-resistant genes and toxin genes by bacteriophages.

Therefore, in order to make bacteriophage therapy socially usable, it has become clear that the issues to be addressed are to improve the accuracy of treatment, to control the target, and to improve safety.

For this reason, attempts are being made to solve these problems using modified bacteriophages. Specifically, it is possible to partially knock-out the bacteriophage genome and make it into a non-proliferative bacteriophage.

For example, it is known that the ability to proliferate can be lost by introducing an Amber mutation and other methods to cause a loss of function in essential genes, as a means of synthesizing non-proliferative bacteriophages. It is also known that the ability to proliferate can be restored by complementing the gene with a plasmid. However, it is thought that the deletion of large sections of the bacteriophage genome and the deletion of multiple genes would be technically difficult, and no such work had been done at all.

In fact, the inventors of the present invention also attempted to synthesize a long gene-deleted bacteriophage by using a plasmid, but this attempt is unsuccessful. Specifically, the bacteriophage's terS gene is deleted and complemented with a plasmid to create a non-proliferative bacteriophage capsid. In this case, non-proliferative bacteriophages are synthesized by using a bacteriophagemid method and a SaPI method, but there are disadvantages such as the need for inducers such as mitomycin C for synthesis, and the small amount of synthesis is the result.

** Furthermore, because non-proliferative bacteriophages do not multiply, it is generally known that they have weak bactericidal activity. In fact, when non-proliferative bacteriophages are used, an exceptionally large dosage is required for treatment because they do not multiply. As in the example described later, when non-proliferative B-CAP only is used on E. coli, almost no bactericidal effect is obtained at an MOI = 1.

In other words, non-proliferative bacteriophages loses the advantage of being able to multiply at the site of infection, and in terms of the large amount of bacteriophages required for actual antibacterial treatment, they are not practical for use in treatment.

For these reasons, it is difficult to use typical bacteriophages and non-proliferative bacteriophages as antibacterial therapeutic agents. The ideal bacteriophage preparation should have the following properties: "efficacy against resistant bacteria," which is effective against resistant bacteria; "high safety", which has little impact on the ecosystem, as in the case of non-proliferative bacteriophages; "mass producibility"; and "strong antibacterial activity," which has a high antibacterial therapeutic effect.

In contrast, B-CAP according to the embodiment of the present disclosure can efficiently utilize because the bacteriophage genome division method makes it possible to incorporate foreign genes into long strands of DNA while maintaining the non-proliferative properties. In other words, by appropriately selecting the exogenous genes to be incorporated and generating B-CAP, it is possible to reliably sterilize bacteria, or the like. For example, while a single bactericidal gene may become resistant due to bacterial evolution, by loading a plurality of genes into B-CAP, this can be suppressed, and the effect can be maintained for a long time.

Further, because B-CAP can target any bacteria for sterilization, depending on the exogenous genes it carries, it has a high "efficacy against resistant bacteria." In other words, B-CAP according to the embodiment of the present disclosure can be an evolutionary adaptive medicine because it can respond to the emergence of new resistant bacteria simply by changing the genes it carries. It is also possible to modify B-CAP itself by using genetic engineering techniques to respond to bacteria.

** Furthermore, B-CAP according to the embodiment in the present disclosure is considered to be superior to typical bacteriophages in terms of safety, as it does not produce self-replicating bacteriophages (replicating bacteriophages or progeny bacteriophages). Also, although there is a problem with typical non-proliferative bacteriophages in that there is a high possibility that a single gene defect could be complemented and replication become possible, B-CAP according to the present embodiment has a defective long bacteriophage genome, and it should be almost impossible for long DNA to be complemented from nature. In other words, B-CAP has an exceptionally low probability of acquiring the ability to proliferate, so it is very safe. Furthermore, B-CAP can only be prepared in bacteria that have been genetically modified to contain the capsid nucleic acid element (capsimid) that synthesizes the divided capsid. In other words, B-CAP cannot be produced in nature, and can only be produced by specialized bacteria that function as a "factory" for B-CAP synthesis.

For these reasons, B-CAP has "high safety" and can solve the biological safety issues of antibacterial bacteriophage preparations.

Furthermore, because B-CAP according to the present embodiment is a synthetic capsid that does not replicate, the effects of B-CAP itself can be suppressed by mutation. For example, because B-CAP does not replicate, it does not incorporate new resistance genes or toxin genes, nor does it spread. In addition, because B-CAP does not replicate, the possibility of prophages being retained in the administered organisms or bacteria in the environment is extremely low, and there is negligible impact on the ecosystem. Further, in principle, it is difficult for resistant bacteria to develop. This enhances safety. Furthermore, because B-CAP does not replicate, it is possible to accurately estimate the administered dose.

In addition, B-CAP according to the embodiment in the present disclosure is derived from natural sources and is therefore safe and has a low environmental impact. This makes it possible to provide safe, environmentally friendly disinfectants and food products.

Further, in terms of "mass production", although the genome assembly of typical non-proliferative bacteriophages uses a complicated method that utilizes yeast, in the case of B-CAP according to the embodiment in the present disclosure, it is possible to perform all of the genome assembly during synthesis in vitro, and mass production can be guaranteed. That means, in the present embodiment, a new concept called the genome division method, which divides the genome of a lysogenic bacteriophage, is used to construct B-CAP that includes the replication region and packaging region necessary for bacteriophage nucleic acid replication, and it is possible to load arbitrary long DNA onto the bacteriophage capsid and synthesize them in large quantities.

By directly assembling the B-CAP genome in vitro, it is possible to synthesize B-CAP with high efficiency, greatly reducing the cost and time required for synthesis. Further, with regard to formulation, since massive quantities (more than approximately 10¹⁰ PFU/ml) of B-CAP can be synthesized cheaply, there are significant manufacturing benefits due to reduced costs.

The B-CAP of the present embodiment has the same infection mode as wild-type bacteriophages, although it is non-proliferative, and can deliver long DNA to target bacteria. Therefore, the B-CAP in the present embodiment can create new modalities because it can be given various biological functions, including bactericidal activity, depending on the nature of the DNA it carries. In other words, B-CAP makes it possible to construct a bacteriophage preparation with high bactericidal activity.

The B-CAP synthesized in the present embodiment is capable of carrying foreign DNA and injecting DNA into target bacteria. The injected DNA can exert biological activity derived from its own DNA, including amplification within the target bacteria.

Further, the bacteriophage genome division method by using capsimid allows the length of the B-CAP DNA sequence that can be maintained within the bacteria to be reduced to about half that of typical bacteriophages. As a result, B-CAP can also function as a cloning vector for long DNA, allowing the maintenance of long, highly toxic genes and the mass synthesis of these genes in a brief period of time. In other words, it is possible to load foreign long DNA onto B-CAP. In fact, in the example described later, we have succeeded in loading 18kb of long DNA derived from Staphylococcus aureus onto B-CAP.

In other words, in addition to its bactericidal effect, or the like, B-CAP can also be used to impart new biological functions.

Here, the B-CAP in the present embodiment can compensate for the disadvantage of non-proliferative bacteriophages, which have weak antibacterial activity because they do not proliferate, by carrying a bactericidal gene that produces a bactericidal substance with strong bactericidal activity even though it is non-proliferative because its product can be secreted to kill the surrounding bacterial group.

For example, by loading a bacteriocin gene that produces a secreted bacteriocin product that kills surrounding bacteria as an exogenous gene into B-CAP, it is possible to construct an antibacterial capsid (NM-ABCapsid) that can kill bacteria around the host bacteria despite being non-proliferative.

In fact, in the example described later, B-CAP_ColE1, which is an NM-ABCapsid, synthesized the secretory bacteriocin (colicin E1) within the target bacteria, and showed an extraordinarily strong mass sterilization effect. That means, B-CAP_ColE1 also kills the bacteria surrounding the target bacteria, and even when administered in the same number as the number of bacteria, it still has a sufficient sterilizing effect. Specifically, according to the example, in vitro experiments showed that the sterilizing effect of B-CAP_ColE1 is 1000 times that of B-CAP alone.

Furthermore, in an infection experiment by using mice, B-CAP_ColE1 showed a remarkable antibacterial therapeutic effect. Specifically, B-CAP_ColE1 has able to treat infection by carbapenem-resistant E. coli, which is a type of resistant bacteria of great clinical importance, in a mouse infection model, and it has been able to keep mice alive from fatal infections. That is, in the example as described later, carbapenem-resistant E. coli has been intraperitoneally administered to mice, and a significant survival effect has been observed in mice infected with the bacteria.

In other words, B-CAP in the present embodiment can enable the construction of a bacteriophage preparation that has a high bactericidal effect, although it is non-proliferative.

Furthermore, although the strong antibacterial activity of bacteriocins is expected to be applied to the treatment of bacterial infections, it is necessary to synthesize and purify massive quantities of bacteriocins for use in treatment. In addition, because bacteriocins exhibit concentration-dependent bactericidal activity, it is necessary to maintain a certain concentration at the site of infection. Therefore, in the creation of antibacterial drugs using bacteriocins, the establishment of a method for mass-producing bacteriocins and the construction of an effective delivery system for bacteriocins to the site of infection are principal issues.

In contrast, B-CAP_ColE1 according to the present embodiment is able to secrete bacteriocin to a level that maintains its concentration at the site of infection while carrying bacteriocin on a safe, non-proliferative bacteriophage by the immune gene, which is a resistance factor that suppresses the sterilization of target bacteria. This allows the antibacterial therapeutic effect of the capsid to be significantly increased.

In addition, in the above-mentioned embodiment, when producing B-CAP, an example of loading the helper B-CAP region of the capsid nucleic acid element (capsimid) into BAC is described.

However, it is also possible to incorporate the capsid nucleic acid element into the genome or plasmids of bacteria. In this case, since general plasmids have a large copy number, it is difficult to maintain bacteriophage genes or long genes that have many toxic genes for the host. For this reason, it is possible to maintain stability by using plasmids with a low copy number, making the toxic bacteriophage genes inducible, inserting long DNA segments into a plurality of plasmids, and the like.

It is also possible to delete non-essential genes from B-CAP or transfer some essential genes to the helper B-CAP side. This makes it possible to load even longer DNA.

Furthermore, when generating a B-CAP for a bacteriophage that is lysogenized on the genome, it is also possible to delete the region required for lysogenization. In this case, it is also possible to leave regions that do not need to be included in the B-CAP (such as virion, or the like) on the chromosome and
to construct only the regions necessary for B-CAP (the replication region necessary for nucleic acid replication and the packaging region), and the like, as B-CAP.

Further, it is also possible to retain the minimum necessary regions of the B-CAP, other than the RNA polymerase, DNA replication region, and packaging region, as the replication region necessary for nucleic acid replication.

Also, in the above-mentioned embodiment, the bacteriophage genome is directly divided when forming B-CAP, but it is also possible to use other sequences.

For example, when constructing B-CAP, it is also possible to use the sequence of a region of about 20kb in size, which is called PICI (Phage-inducible chromosomal island) and is contained in the genome of the bacterium. In the case of Staphylococcus aureus, the PICIs is also called SaPI (Staphylococcus aureus pathogenicity islands.)

PICIs or SaPIs are similar to lysogenic bacteriophages, which can introduce the bacterial genome, but they do not have the genes that make up the bacteriophage virion. For this reason, the genome length is about half that of a lysogenic bacteriophage. Despite its compact structure, PICI can be induced and replicated from the genome during bacteriophage infection, and the genome can be packaged within the bacteriophage capsid. This packaging requires a packaging sequence derived from PICI, and when this region is loaded onto a plasmid, the plasmid can be inserted into the lysogenic bacteriophage.

In addition, PICI that has entered the bacteriophage capsid is known to be transduced into other bacteria in the same way as bacteriophages, and since PICI is transmitted via bacteriophages, it can be used in B-CAP.

Further, it is also possible to construct a system that uses the DNA of this PICI or SaPI together with the DNA of the bacteriophage.

Furthermore, although the above-mentioned embodiment uses T7 bacteriophage as the basis for creating B-CAP, it is also possible to construct it using other bacteria or bacteriophages other than T7.

For example, it is possible to use T4 bacteriophage, a model bacteriophage that is approximately 170kb in size, or other jumbo bacteriophages to carry longer DNA.

Furthermore, the inventors of the present invention have produced B-CAPs by using E. coli bacteriophage T7 as an example of a Gram-negative bacterium and Staphylococcus aureus bacteriophage Tan2 as an example of a Gram-positive bacterium. In other words, bacteriophage preparations by using B-CAPs can be applied to a wide range of bacterial species.

Also, in the above-mentioned embodiment, we have described an example of using a general bacteriophage for B-CAP. However, it is also possible to use viruses, RNA viruses, bacteriophagemids, or the like, which are different from general bacteriophages, as B-CAP. In addition, B-CAP may be provided in the form of a prophage integrated into the bacterial genome or plasmid. Also, for example, bacteriophagemid can be introduced into cells via any method, such as electroporation and nanoparticles, in the bacterial discrimination method of the present embodiment.

In the above-described embodiments, examples are given by using a bactericidal gene, a biofilm-degrading gene, an antigen-presenting gene, and a gene for introduction as the exogenous gene.

However, the exogenous gene can be any gene. Furthermore, the exogenous gene may include a gene that is expressed, regulated, or associated with a drug-resistance gene or a toxin gene. In addition, the exogenous gene may include a pathogenic gene related to other pathogenicity and a gene that suppresses it.

Furthermore, the exogenous gene of the present embodiment may be a broad sense "gene" such as a sequence of gene mutation, single nucleotide substitution, repeat, or the like, which is a target of cancer treatment or hereditary disease. In this case, it is also possible to administer the bacteria themselves as a drug carrier for therapeutic use. In other words, this can also be used for applications such as including the drug corresponding to the exogenous gene in the cytoplasm of the target bacteria, or the like, and then dispersing it around the tissue or target cell by lysing the bacteria. In this case, it is also possible to provide the bacteria themselves that contain the prophage. It is also possible to include a prophage that is activated by a specific immune response.

Also, in the above-mentioned embodiments, an example by using B-CAP as a treatment composition for the treatment of infectious diseases is described.

However, the treatment can be used not only for the treatment of infectious diseases themselves, but also for modifying the bacterial flora, pre-processing for fecal transplantation, or the like. Specifically, it is also possible to induce immunity to bacteria that are therapeutically necessary within the bacterial flora by using antigen-presenting genes as exogenous genes.

Furthermore, as the FDA (U. S. Food and Drug Administration) has approved the use of bacteriophages as a food additive, B-CAP can also be used to sterilize food and prevent corrosion.

In addition, B-CAP according to the embodiment in the present disclosure can also be used in combination with other compositions, or the like. Furthermore, it can also be provided as a "cocktail" containing multiple types of B-CAP. The composition according to the present disclosure may also be administered, sprayed, applied, or the like, at the same time as other compositions.

### [EXAMPLES]

The following sections describe the construction (production), sterilization (bacterial removal), and animal treatment methods for B-CAP in the form of specific example based on specific experiments. However, the Example is just one example and is not limited to this.

### [Method]

### (Bacterial strains and culture conditions)

The strains used in this example are shown in Table 1 below. The strains are grown in LB medium (BD Difco) at 37°C. Unless otherwise specified, appropriate antibiotics are added to the growth medium at the following final concentrations: 100 ug/ml for ampicillin (Amp), 30 ug/ml for kanamycin (Km), and 34 ug/ml for chloramphenicol (Cm).

**[Table 1]**

| Bacterial strain | Description |
|---|---|
| MC1061 | hsdR, mcrB, araD139, Δ(araABC-leu)7679, ΔlacX74, galU, galK, rpsL, thi |
| MC1061 (pKLC172) | MC1061 transformed with pKLC172 |
| MC1061R | MC1061 ColE1-resistant |
| HST08 | F-, endA1, supE44, thi-1, recA1, relA1, gyrA96, phoA, Φ80dlacZΔ M15, Δ(lacZYA-argF)U169, Δ(mrr-hsdRMS-mcrBC), ΔmcrA, λ- |
| Ec89, JMUB4401 | E. coli_CPE/CRE, isolated in Jichi Medical University |
| Ec93, JMUB4405 | E. coli_CPE/CRE, isolated in Jichi Medical University |
| Ec101, JMUB4413 | E. coli_CPE/CRE, isolated in Jichi Medical University |
| Ec320, JMUB5853 | Escherichia coli. ESBL |
| Ec353, JMUB5886 | Escherichia coli, ESBL |
| Ec118, JMUB4430 | E. coli_CPE/CRE, isolated in Jichi Medical University |
| USA300 | *Methicillin-resistant S. aureus (MRSA), FPR3757* |

Among these, MC1061 and USA300 are manufactured by Lucigen. HST08 is manufactured by Takara Bio. ESBL (Extended Spectrum β-Lactamase) E. coli strains Ec320 and Ec353 are obtained from Dr. Zenzo Nagasawa.

### (DNA used)

The DNA used in this example, including plasmid vectors and BAC (Bacterial Artificial Chromosome) DNA, is shown in Table 2 below.

**Table 2**

| Vectetor | Purpose | Discription |
|---|---|---|
| pKLC23 | Arabinose-inducible target gene expression vector | Antibiotics resistance gene expression vector, p15A ori, PBAD promoter, CAT |
| pKLC146 | Arabinose-inducible ColE1 gene expression vector | ColE1 expression vector under the control of anhydrotetracycline-inducible promoter, p15A ori, CAT |
| pKLC172 | Helper B-CAP | Virion genes, BAC ori, CmR |
| pKLC186 | B-CAP | T7 phage without virion genes |
| pKLC187 | Plasmid vector carrying ColE1, immunity protein and release protein | pKLC146 without PBAD promoter, T7 phage-derived sequence is inserted for B-CAP(ColE1) synthesis, p15A ori, CAT |
| pKLC188 | B-CAP (ColE1) | B-CAP carrying ColE1 gene, immunity protein and release |
| pSMART BAC BamHI | Construction of Helper B-CAP | BAC ori, CAT |
| ColE1 DNA | Construction of pKLC146 | ColE1 plasmid purified from E. coli JC411 |

### (Primers used)

The primers used in this example are shown in Table 3 and the nucleotide sequence table below.

**[Table 3]**

| Purpose | | No. | Primer name | Sequence |
|---|---|---|---|---|
| Construction of pKLC172 | Fragment PCR Figure 1 | 1 | T7InF172 T7 6.5-12 clo 8738bp-f | taaactcaaggtccctaaattaatacgactcactataggg |
| | | 2 | T7InF172 T7 6.5-12 clo 8738bp-r | ccacagggagaatatttaattaaataccggaacttctccg |
| | | 3 | T7InF172 T7 13-17 clo 9108bp-f | cggagaagttccggtatttaattaaatattctccctgtgg |
| | | 4 | T7InF172 T7 13-17 clo 9108bp-r | ctacacgtctttccttgtgatttaccaattactcgttctc |
| | | 5 | T7InF172 pSMART BAC 7495bp-f | gagaacgagtaattggtaaatcacacaaggaaagacgtgtagaatcataccaacatggtcaaataaaacgaaaggctc |
| | | 6 | T7InF172 pSMART BAC 7495bp-r | ccctatagtgagtcgtattaatttagggaccttgagtttactaaatactagtgactccagcgtaactggactg |
| | Colony PCR | 7 | pKLC172 check primers 302bp det1-f | CTTTCTCTGTCCTTCCTGTG |
| | | 8 | pKLC172 check primers 302bp det1-r | cthhaatatcgttagggttc |
| | | 9 | pKLC172 check primers 284bp det2-f | acaatatcgattccctgtgg |
| | | 10 | pKLC172 check primers 284bp det2-r | tcaagaatgtcatggtgagc |
| | | 11 | pKLC172 check primers 316bp det3-f | cttcatagcctctgaatggtg |
| | | 12 | pKLC172 check primers 316bp det3-r | TTTAGCTTCCTTAGCTCCTG |
| Construction of B-CAP(pKLC186) | Fragment PCR Figure 1 | 13 | B-CAP frag1-f | cttagccagaataatcaagtcgttacacgacactaagatccacggatggactctcaaggaggtacaaggtgc |
| | | 14 | B-CAP frag1-r | gctttcacttcctcaaaccagtcgttgatgtgc |
| | | 15 | B-CAP frag2-f | tgattgcacgcatcaacgactggtttgaggaagtg |
| | | 16 | B-CAP frag2-r | caactgctttgaaaccttgtcctctaggccagagc |
| | | 17 | B-CAP frag3-f | gtttcgctctggcctagaggacaaggtttcaaagc |
| | | 18 | B-CAP frag3-r | tcacttagtgtcgtgtaacgacttgattattctggctaagtcc |
| | Confirmation by PCR Figure 5 | 19 | B-CAP det1 274bp-f | ccaagcctctcatcactacc |
| | | 20 | B-CAP det1 274bp-f | tgcttagcttcaaggtcacg |
| | | 21 | B-CAP det2 378bp-f | tatggtgtctcccaacagac |
| | | 22 | B-CAP det2 378bp-r | ccataaracgttggagaacc |
| | | 23 | B-CAP det3 505bp-f | ttacctgtggagaccgtagc |
| | | 24 | B-CAP det3 505bp-r | aacttgtggcggtctaacag |
| | | 25 | T7 check det1 9022bp-f | tageggataactcaagggtalcgcaaggtg |
| | | 26 | T7 check det1 9022bp-r | tttctcccgcagttccacttagaagtctcc |
| | | 27 | T7 check det2 9013bp-f | tgaagtatcacgaggcttatgctgctg |
| | | 28 | T7 check det2 9013bp-r | cgagccatttggccctgcttgataatatcc |
| | | 29 | T7 check det3-mini3(det6) 8456bp mini5221bp-f | tgtaccactggtaacatcctgactcagacc |
| | | 30 | T7 check det3 8456bp-r | ccaaagtcccaatgagaccacgactgttgc |
| | | 31 | T7 check det4 8598bp-f | cttccgtaaccgcttaggattccttagtgg |
| | | 32 | T7 check det4 8598bp-r | aagctgttgagtcaatggetcgttcggtac |
| | | 33 | T7 check det5 7009bp-f | tttctatgctatggctgcacacgtcaaagc |
| | | 34 | T7 check det5-mini3(det6) 7009bp mini5221bp-r, det6 as 23,027bp | ttcatcctcctcgtactctcgacttcctc |
| Construction of B-CAP(S. aureus DNA) | Fragment PCR Figure 8 | 35 | InF203 pKLC186 frag1-f | aagccgtgtctttcgtagagtctgccattaagaag |
| | | 36 | InF203 pKLC186 frag1-r | tcatggtacttaacgtcctccagttcagcttccag |
| | | 37 | InF203 pKLC186 frag2-f | ctaaactggaagctggaggacgttaagtac |
| | | 38 | InF203 pKLC186 frag2-r | ataaatcaccactcaatgaaagacgtaccgtaacc |
| | | 39 | InF203 USA300_17058 frag3-f | ggttacggtacgtctttcattgagtggtgatttatgtttaatcgcgacgtacgagtagaaaagctagc |
| | | 40 | InF203 USA300_18010 frag3-f | ggttacggtctttcattgagtggtgatttataaaggagaaagagatgacgttagaacgcgtgaaac |
| | | 41 | InF203 USA300_19002 frag3-f | ggttacggtacgtctttcattgagtggttgatttatcctcaacacaaaaccaactcacacagcaacgttc |
| | | 42 | InF203 USA300_20020 frag3-f | ggttacggtacgtctttcattgagtggtgatttatttcgatgttgaatcgatgttgaatcgtttaacgtgtcacatgatgc |
| | | 43 | InF203 USA300_frag3-r | tgactaaccgaagaagtcgtgtcagatacatttcg |
| | | 44 | InF203 USA300_frag4-f | tgaatcgaaatgtatctgacacgacttcttcggtt |
| | | 45 | InF203 USA300_frag4-r | gtggtctatagtgcatccctatgcagtcctaatgctgggatttctgccttagtgacaaagttttctgaagc |
| | | 46 | InF203 pKLC186 frag5-f | gcattaggactgcatagggatgcactatagaccac |
| | | 47 | InF203 pKLC186 frag5-r | agagccttcttaatggcagactctacgaaagacac |
| | PCR check Figure 11 | 48 | pKLC203 insert check1-f | atgcccttgcgttaggcattgagtatctcc |
| | | 49 | pKLC203 insert check1-r | gctccaacatgaagatggctatcgtgtcac |
| | | 50 | pKLC203 insert check2-f | ctgtttgagggtggatagcagtacctgagc |
| | | 51 | pKLC203 insert check2-r | aaatcggtaggtgactctatgccgcagtag |
| | sequence Figure 12 | 52 | pKLC203 det-up-f | agtccatgcagttggattcc |
| | | 53 | pKLC203 det-down-f | ttcaagtgcgtttcgtgtgg |
| Construction of B-CAP(ColE1) | Construction of pKLC 146 and pKLC187 ColE1 expression plasmid | 54 | InF146-148 ColE1 clo 2097bp-f | atggaaccgcggtagcgtac |
| | | 55 | InF146-148 ColE1 clo 2097bp-r | cttttctactgaaccgcgatcc |
| | | 56 | InF146 pKLC23R-r | tacgctaccgcggtttccatGGATCCtttctcctggtacc |
| | | 57 | InF146-147 pKLC23R-f | atcgcggttcagtagaaaaggttttggcggatgagagaag |
| | | 58 | InF187 pKLC146-f | ggagataggggcctttacgattattactttaagatttaactctaagaaggaggagatatacatatggaaaccgcggtagcgtactataaagatgg |
| | | 59 | InF187 pKLC146-r | tcgtaaaggcccctatctccctatagtgagtcgtattaatttagggaccttgagtttagataagctgtcaaacatgagcagatcctctacg |
| | Sequence primer for pKLC187 | 60 | Seq1 pKLC187 | gctgcattattagcatgagc |
| | | 61 | Seq2 pKLC187 | cgaaagcaaaggcaaacagg |
| | | 62 | Seq3 pKLC187 | tcaccaagagccaatgatcc |
| | | 63 | Seq4 pKLC187 | cggtgaagtatgatgactgg |
| | | 64 | Seq5 pKLC187 | gcatcttatctgacacaagg |
| | Fragment PCR | 65 | B-CAP_ColE1 frag1-f | tccacggatggactctcaaggtacaaggtgc |
| | | 66 | B-CAP_ColE1 frag1-r | gctttcacttcctcaaaccagtcgttgatgcgtgc |
| | | 67 | B-CAP_ColE1 frag2-f | tgattgcacgcatcaacgactggtttgaggaagtg |
| | | 68 | B-CAP_ColE1 frag2-r | caactgctttgaaccttgtcctctaggccagagc |
| | | 69 | B-CAP_ColE1 frag3-f | gtttcgctctggcctagaggacaaggtttcaaagc |
| | | 70 | B-CAP_ColE1 frag3-r | tcacttagtgtcgtgtaacgacttgattattctggctaagtcc |
| | | 71 | B-CAP_ColE1 pKLC187 frag4-f | cttagccagaataatcaagtcgttacacgacactaagtgtgataaactcaaggtccctaaattaatacgactcactatagggag |
| | | 72 | B-CAP_ColE1 pKLC187 frag4-r | atgatagcaccttgtacctccttgagagtccatccgtggactactgaaccgcaatccccgtcag |

### (Helper B-CAP construct)

The regions from gp6.5 to gp12 (8.7 kb) and from gp13 to gp17 (9.1 kb) are amplified from T7 bacteriophage DNA using the two-step method with KOD Fx Neo (Toyobo Co.). In addition, the region from the rrnBT1 terminator to the TTe terminator (7.5 kb) is amplified in the same way. The amplified PCR fragments are gel-extracted by using the FastGene Gel/PCR Extraction Kit (NIPPON Genetics) and the concentration is measured. Each fragment of 100 ng is made into a total of 20 µl with 10 µl of NEBuilder (New England Biolabs), and then combined at 50°C for 1 hour. Then, we prepared electrocompetent cells of MC1061 E. coli. Firstly, 100 ml of MC1061 E. coli in the logarithmic growth phase with an OD₆₀₀ = 0.5 is left to stand on ice for 20 minutes, and then it is washed three times with ice-cold 10% glycerol. The cells are finally suspended in 200 to 400 µl of 10% glycerol. 5 µl of DNA is added to 50 µl of electrocompetent cells, transferred to an ice-cooled 1 mm cuvette, and electroporated by using ELEPO21. The electroporation conditions are as follows: Poring Pulse voltage: 1,500V, pulse width: 2.5ms, pulse interval: 50ms, number of times: 1, polarity: +; and Transfer Pulse Voltage: 150V, Pulse Width: 50ms, Pulse Interval: 50ms, Number of Pulses: 5, Polarity: +/-. After that, the electroporated E. coli is placed in 1 ml of SOC medium and incubated at 37°C for 30 minutes, and 50 µl is spread on an LB plate containing chloramphenicol. Eight colonies are obtained, and the strain with the highest efficiency for B-CAP synthesis is selected as the E. coli strain that harbored helper B-CAP (hereinafter referred to as "MC1061 (pKLC172)" strain). In addition, the MC1061R strain, which is resistant to ColE1, is similarly electroporated with pKLC172 to form the "MC1061R (pKLC172)" strain. The presence of pKLC172 in these strains is confirmed by using PCR and deep sequencing. Also, MC1061 (pKLC172) strain or MC1061R (pKLC172) strain is an example of the bacteria used in the tests as described later. In other words, the MC1061 (pKLC172) strain or MC1061R (pKLC172) strain is used as "propagation strains" for the growth and production of B-CAP or B-CAP _ColE1.

The helper B-CAP for Staphylococcus aureus is prepared by dividing it into two plasmids. The plasmid pLC-tailTan2 is constructed by making the tail portion of the lysogenic bacteriophage of Staphylococcus aureus tetracycline-inducible, and the plasmid pNL-headTan2 is constructed by making the head portion cadmium-inducible. The drug resistance of each is used with chloramphenicol and erythromycin.

The primer sequences used in the PCR are listed in the above Table 3 and the sequence list.

### (Generation of B-CAP)

In the case of B-CAP for E. coli, the regions from gp17.5 to gp1 (6.8 kb), from gp1 to gp2.8 (6.9 kb), and the region from gp3 to gp6.3 (8.2 kb) in DNA of T7 bacteriophage are amplified, respectively, by using the two-step method with KOD Fx Neo (Toyobo). The amplified PCR fragments are gel-extracted by using the FastGene Gel/PCR Extraction Kit (NIPPON Genetics) and their concentrations are measured. 100 ng of each fragment is made into a total of 20 µl with 10 µl of NEBuilder (New England Biolabs) and assembled at 50°C for 4 hours. The MC1061 (pKLC172) strain, which contains helper B-CAP, is cultured in LB medium containing chloramphenicol, and when it becomes OD₆₀₀=0.5, the E. coli cells are cooled and washed three times with ice-cold 10% glycerol. The cells are suspended in ice-cold 10% glycerol to a concentration of about 500 times that at the time of shaking, and it is used as electrocompetent cells. 5 µl of the prepared B-CAP reaction solution is added to 50 µl of the electrocompetent cells, and the mixture is transferred to an ice-cooled 1 mm cuvette. Electroporation is performed under the same conditions as for the preparation of B-CAP, and the cells are incubated in 1 ml of SOC medium at 37°C for 30 minutes. After that, the entire culture product is mixed with 3 ml of LTA (LB 0.5% agarose containing 1 mM CaCl₂, 56°C) and layered on an LB plate. After having the LB plate dried for about 10 minutes, B-CAP plaques appeared after about 8 hours in an incubator at 37°C. The produced B-CAPs are confirmed by using PCR and deep sequencing.

The B-CAP of Staphylococcus aureus is constructed by electroporation of B-CAP DNA into the RNA4220 strain, which contains helper B-CAP DNA. 150 µl of the overnight culture is added to 4 ml of TSA top agar medium containing erythromycin (15 µg/ml), chloramphenicol (15 µg/ml), CaCl2 (2.5 µM), and ATc (1 µg/ml), mixed, and then poured onto a TSA plate. This plate is incubated overnight in a 37°C incubator.

The primer sequences used in the PCR are listed in the above Table 3 and the sequence list.

### (Generation of B-CAP carrying the exogenous gene)

Firstly, we prepared the DNA of the helper B-CAP and B-CAP. In order to insert the exogenous gene into the B-CAP, we made three fragments of the B-CAP by PCR and amplified the exogenous gene by PCR. We performed PCR on Staphylococcus aureus and ColE1 as exogenous genes. The template for each PCR is Staphylococcus aureus genomic DNA and pKLC187. We added homologous sequences to the ends of each fragment so that they could be assembled by using NEBuilder. Using these homologous sequences, the four gene fragments are assembled by using NEBuilder. The assembled DNA and Helper B-CAP are electroporated into HST08 (Takara), which is a competent cell of E. coli. The electroporation conditions are the same as those described above. The electroporated product is transferred to 1 ml of SOC medium and shaken at 37°C for 30 minutes. 1 ml of MC1061 (pKLC172), which is an E. coli strain containing helper B-CAP, is added, and 8 ml of LB medium containing 1 mM CaCl2 is also added. After incubating at 37°C for 30 minutes, 1 ml is taken from the approximately 10 ml of culture fluid and mixed with 3 ml of LTA (LB 0.5% agarose containing 1 mM CaCl₂, 56°C), and then layered on LB plates containing chloramphenicol. After allowing the plates to dry for about 10 minutes, they are incubated in an incubator at 37°C.

The primer sequences used in the PCR are listed in the above Table 3 and the sequence list.

### (Preparation of Staphylococcus aureus and ColE1 sequences)

The DNA sequences of Staphylococcus aureus (methicillin-resistant S. aureus, MRSA) and ColE1 are prepared for loading onto the B-CAP. The mixture is shaken at 37°C for 20 minutes, and then centrifuged. The pellet is suspended in 1 ml of TE buffer. Then, 10 µl of Achromopeptidase (50 U/ul), 5 µl of lysostaphin (10 mg/ml) and 1 µl of RNase are added, and the mixture is incubated at 37°C for 2 hours incubated at 57°C for 2 hours, and 1.2 ml of saturated phenol in TE is added and mixed well. After centrifugation at 12,000 x g for 10 minutes, the supernatant is transferred to a new tube. The same amount of chloroform and isoamyl alcohol (25:1) as the sample is added and mixed, and the mixture is centrifuged again at 12,000 x g for 10 minutes. The supernatant is mixed with 2.5 times the volume of ethanol and 1/10 of 3M sodium acetate, cooled at -20°C, and then centrifuged. Finally, it is washed with 70% ethanol and used as a DNA sample for Staphylococcus aureus. The ColE1 gene sequence is obtained by amplifying the ColE1 gene cassette (including immune gene and release gene) from ColE1 DNA (NIPPON GENE) by PCR, and it is cloned into pKLC23, which is an arabinose-inducible expression vector as described in Non-Patent Document 2, and named pKLC146. pKLC146 is designed so that ColE1 is located immediately downstream of the arabinose-inducible promoter, and the immune gene and release gene are aligned downward.

The pKLC146 plasmid is transformed into E. coli MC1061, and after 12 hours of incubation in LB medium containing 0.2% L-arabinose, the supernatant is collected. After centrifugation at 5,000 x g for 10 minutes, the supernatant is filtered through a 0.22 µm filter. This filtrate is added to MC1061, and bacterial growth is strongly suppressed. In other words, we confirmed that ColE1 is being synthesized from pKLC146. Subsequently, in order to facilitate the incorporation of ColE1 into B-CAP, a plasmid is generated by inserting a partial sequence of T7 bacteriophage into pKL146 and is named pKL187.

The primer sequences used in PCR are listed in the above Table 3 and the sequence list.

### (Preparation of T7 bacteriophage-resistant E. coli)

200 µl of E. coli MC1061 strain cultured overnight is mixed with 3 ml of LTA (LB 0.5% agarose containing 1 mM CaCl₂, 56°C), and the mixture is layered on an LB plate. After drying for about 10 minutes to allow the agarose to set, 5 µl of T7 bacteriophage solution (10⁸ PFU (Plaque Forming Unit) / ml) is dropped on top of the LTA. After 20 hours of incubation at 37°C, the colonies that appeared are collected and spread on a new LB plate. The colonies that appeared are spread again on a new LB plate, and the colonies that appeared are cultured in LB medium. The colonies are confirmed to be resistant to T7 bacteriophage by using an LTA agar experiment, and they are stored.

### (TEM imaging of bacteriophage and B-CAP)

A drop of concentrated bacteriophage suspension is first dropped onto a 400-mesh copper grid coated with collodion (Nissin EM Co., Ltd., Japan), and the bacteriophage is allowed to adsorb for 10 minutes. After that, the residual liquid is removed with filter paper, and 1% uranyl acetate (negative staining solution) is dropped onto the copper grid. The mesh grid is completely soaked for about 5 seconds, and then dried by blotting. After staining, it is observed by using an 80kV transmission electron microscope (TEM) HT7700 (Hitachi). Transmission electron micrographs of the bacteriophage are taken by using an 8-megapixel main digital camera.

### (DNA extraction from bacteriophage and B-CAP)

The target bacteriophage and B-CAP are added to the logarithmically growing host cells at a multiplicity of infection (MOI) of 0.1 to 0.01, and the mixture is incubated at 37°C until the turbidity of the solution became faint. The supernatant is then centrifuged at 5,000 g for 10 minutes, and the supernatant is collected in a new tube. Add 10 µg/ml DNase (Sigma-Aldrich) and 10 ug/ml RNase (Sigma-Aldrich) to the final concentration, and stir at 37°C for 1 hour. Add an equal volume of PEG solution (10% (w/v) PEG 6000, 5 mM Tris-HCl (pH 7.5), 1 M NaCl, 5 mM MgSO₄·7H₂O) is gradually added. After incubating at 4°C overnight, the mixture is centrifuged at 15000 g for 30 minutes at 4°C, and the precipitated bacteriophage is collected. The bacteriophage pellet is resuspended in TE buffer (10 mM Tris-HCl (pH 8), 1 mM EDTA), and 1/100 volume of 1 M EDTA and 1/100 volume of 10% SDS are added to the bacteriophage suspension, and it is incubated at 68°C for 15 minutes. After incubation, an equal volume of phenol : chloroform : isoamyl alcohol (25:24:1, v/v/v) is added and the solution is mixed well. After centrifugation, the aqueous phase is transferred to a new tube, and an equal volume of chloroform : isoamyl alcohol (24:1, v/v) is added and mixed well again. After centrifugation, the aqueous phase containing the bacteriophage DNA is transferred to a new tube, and 2.5 times the volume of ethanol is added. After incubating at -20°C for 1 hour, the mixture is centrifuged at 12,000 g for 10 minutes at 4°C. The DNA pellet is washed twice with 1 ml of 70% ethanol, and the DNA is lightly dried and then resuspended in an appropriate amount of TE buffer.

### (Plate-based bacterial growth inhibition test)

100 µl of bacteria that had been cultured overnight is mixed with 3 ml of LB soft agar (LB, 0.5% agarose, 1 mM CaCl, 1 mM CaCl₂, 56°C), and it is quickly poured into a round-shaped LB plate. If using a square-shaped plate, double the pouring volume. After leaving the plate at room temperature for 5 minutes, 2 µl of B-CAP or bacteriophage is directly spotted onto the upper agar containing bacterial cells, and the plate is incubated at 37°C. After approximately 6 hours of incubation, the formation of plaques is observed and photographed.

### (Inhibition of bacterial growth by culture fluid)

The MC1061 strain or the MC1061R strain, which is resistant to ColE1, is plated on LB plates and incubated at 37°C for 12 hours. The plates are transferred to LB medium containing colonies on the plates and incubated at 37°C for 8 hours. After confirming that the bacteria had grown sufficiently, each culture is diluted 100-fold with LB medium and shaken vigorously (400 rpm) at 37°C for 4 hours. When the OD (650 nm) reached approximately 0.5, the culture is started again by adjusting the OD₆₀₀=0.25 with LB medium. After 2 hours of incubation, B-CAP/B-CAP (ColE1) is added to the growing E. coli solution at approximate MOI=0.001, 0.01, 0.1, and 1 (10⁶, 10⁷, 10⁸, and 10⁹ PFU/ml,) and the shaking culture is continued for another 24 hours. During this time, the turbidity of the bacteria is measured every hour.

### (Measurement of bacteriophage titers)

T7 bacteriophage and B-CAP are diluted 10-fold in SM buffer in the range of 10⁻⁴ to 10⁻⁷. On the other hand, the overnight culture of E. coli strains MC1061 and MC1061 (pKLC172), which had been diluted 1:100 in LB culture medium, is cultured at 37°C with stirring until the OD₆₀₀ reached approximately 0.5. After that, 10 µl of each dilution of T7 bacteriophage/B-CAP is added to 100 µl of the bacterial suspension, and the mixture is incubated at 37°C for 20 minutes. Then, the entire volume of the culture is mixed with 0.5% LTA containing 1 mM CaCl₂ and poured onto the surface of an LB plate. The plates are then incubated at 37°C overnight. The number of plaques on the plates is counted, and the PFU/mL is calculated as the titer of the bacteriophage.

### (Whole genome sequencing of extracted DNA)

The extracted DNA is sequenced by using a next-generation sequencer. Firstly, each genomic DNA is randomly sheared into short fragments, and the obtained fragments are repaired at the ends, A-tailed, and ligated with Illumina adapters. The fragments with adapters are amplified by PCR, and the size is selected and purified. The library is quantified by using Qubit and real-time PCR, and the size distribution is detected by using a bioanalyzer. Nextera XT Library Prep Kit (Illumina) is used to construct a paired-end sequencing library from the plasmid. Sequencing is conducted on the Illumina MiSeq platform (2×301bp) by using the MiSeq reagent kit version 3 (Illumina). The number of reads is 7,059,496 and 8,277,786 for B-CAP, 8,000,044 and 8,691,738 for B-CAP (ColE1), and 8,814,734 and 8,173,908 for pKLC172. The read sequences are trimmed and assembled de novo into contigs by using CLC Genomics Workbench (Qiagen). CLC Genomics Workbench is used to evaluate sequence errors and DNA circularization, and RAST ver. 2.038 is used to annotate the obtained genome.

### (Mouse survival test)

To evaluate the effect of B-CAP/B-CAP_ColE1 on the treatment of E. coli infection, 7-week-old Balb/c (Clea, Japan) mice are used in the survival test. The mice are kept in the laboratory for one week before the assay to acclimate them to the laboratory environment. Firstly, an overnight culture of carbapenem-resistant Escherichia coli is diluted 1:1000 with fresh LB medium and then incubated at 37°C with agitation until the OD₆₀₀ reached approximately 0.5. After that, the bacterial cells are washed twice with PBS and adjusted to a density of approximately 1×10¹⁰ CFU/mL with PBS. After PEG precipitation, B-CAP, B-CAP (ColE1), and T7 bacteriophage are dissolved in SM buffer and dialyzed in PBS for one day. The titers of each are measured by using MC1061 or MC1061 (pKLC172).

After the mice are anesthetized, 6 mice are selected from each group and 200 µl of bacterial suspension (2×10⁹ CFU/mouse) is injected directly into the abdominal cavity. After re-anesthetizing the mice and 1.5 hours later, 200 µl of PBS containing B-CAP, B-CAP (ColE1), T7 bacteriophage, or PBS alone is injected into the same site where the bacterial suspension is injected. A mixed anesthetics of medetomidine hydrochloride, midazolam, and butorphanol tartrate is injected subcutaneously into the back of the mouse at a dose of 200 µl, and the mouse is then awakened from anesthesia with atipamezole, and survival is observed for up to 5 days. The cumulative mortality rate and body weight at 48 and 72 hours after infection are recorded for each treatment group. Kaplan-Meier survival curves are then generated from the data of the three independent experiments and analyzed by using the log-rank test with the Prism 8 software.

### [Results]

### (Construction of B-CAP using bacteriophage genome partitioning method)

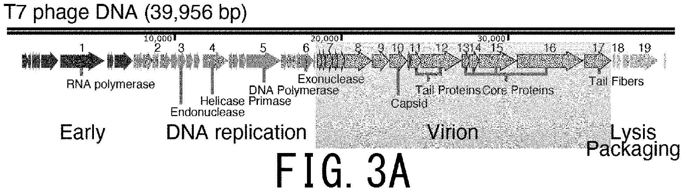

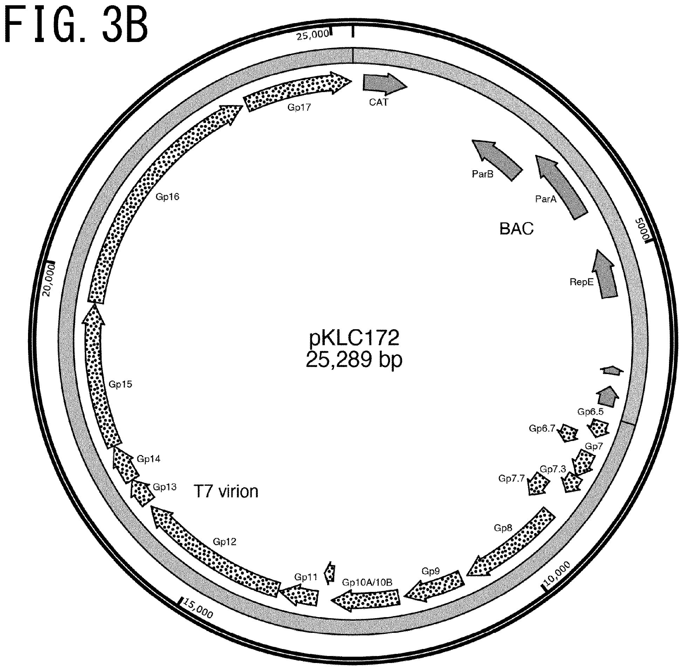

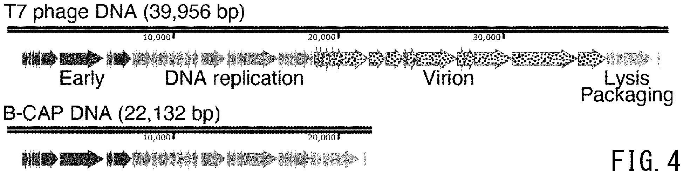

As refer to FIG. 3A, 3B, and 4, the construction (synthesis, production) of B-CAP is described.

FIG. 3A shows the results of extracting the DNA of the T7 bacteriophage, which is a model of a lysogenic bacteriophage, reading the sequence by using a next-generation sequencer, and annotating it by using RAST ver. 2.0. The sequence length of this T7 bacteriophage is 39,956 bp.

In this example, the T7 bacteriophage genome is divided into two parts for B-CAP synthesis: B-CAP DNA, which is the B-CAP element, and Helper B-CAP DNA, which is the capsid nucleic acid element (capsimid). Each divided DNA is assembled, respectively.

Among these, the B-CAP DNA includes regions that have roles for Early genes (Host-virus interaction and RNA polymerase), DNA replication, lysis, and packaging.

The helper B-CAP DNA for constructing B-CAP is designed to include the virion structural region. In order to maintain this helper B-CAP DNA stably within the bacteria, it is cloned into a BAC (Bacterial Artificial Chromosome) to generate pKLC172.

FIG. 3B shows an example of pKL172, which is produced by cloning the virion structural gene region into a BAC as a helper B-CAP in the present example. This pKLC172 is a combination of the BAC replication system and all of the virion structural genes of the T7 bacteriophage. The sequence of pKLC172 is read by using the next-generation sequencer, and it is confirmed to be 25289 bp. Furthermore, we used RAST ver. 2.0 to perform annotation.

Then, as refer to FIG. 4, we explain the sequence structure of the generated B-CAP.

B-CAP DNA is electroporated into E. coli HST08, which has helper B-CAP DNA, and B-CAP plaques are formed. After isolating the plaques, we investigated whether B-CAP is constructed. Specifically, the genome DNA of T7 bacteriophage and B-CAP is extracted, the sequence is read by using a next-generation sequencer, and the sequence is annotated by using RAST ver. 2.0.

As a result of examining the whole genome sequence, B-CAP is 22,132 bp in length, and it retained the Early gene, DNA replication, Lysis, and Packaging regions, but the virion genes are missing.

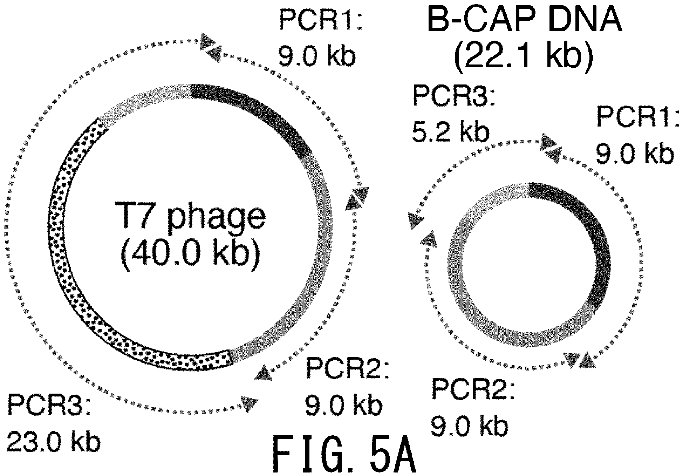

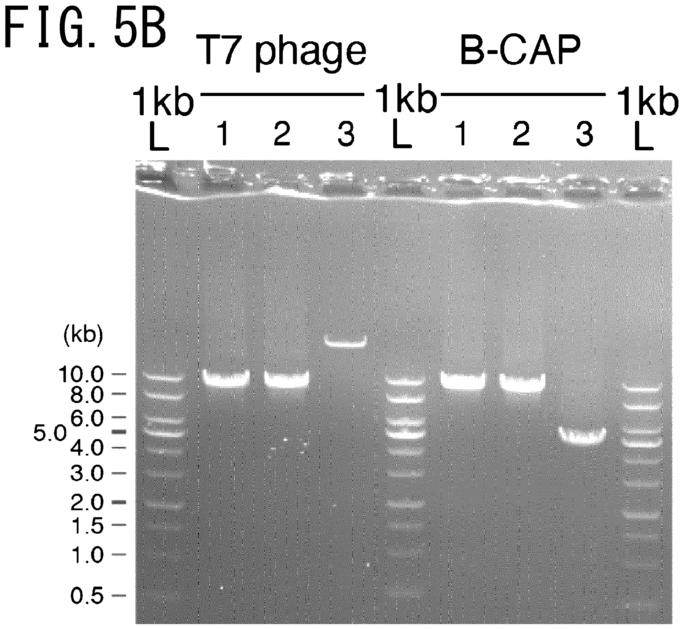

As refer to FIG. 5A and 5B, the results of confirming the construction of B-CAP by using the purified T7 bacteriophage and B-CAP DNA as templates in a PCR reaction is explained.

FIG. 5A shows the configuration of the primers used to confirm the T7 bacteriophage and B-CAP. Specifically, PCR is performed by using the respective primer sets, "B-CAP det1 274bp-f" to "T7 check det5-mini3 (det 6) 7009bp mini5221bp-r, det6 as 23,027bp" as shown in the Table 3 above are used for PCR.

FIG. 5B shows the results of electrophoresis on a 0.5% agarose gel. This confirmed that the size of the B-CAP genome DNA had been shortened to approximately 22 kb. It is also confirmed that the length of the PCR (PCR3) fragment for the virion, lysis, and packaging regions of the T7 bacteriophage is 23.0 kb and the length of the corresponding region of B-CAP is 5.2 kb.

Then, T7 bacteriophage and B-CAP are purified, negatively stained, and photographed by using an electron microscope.

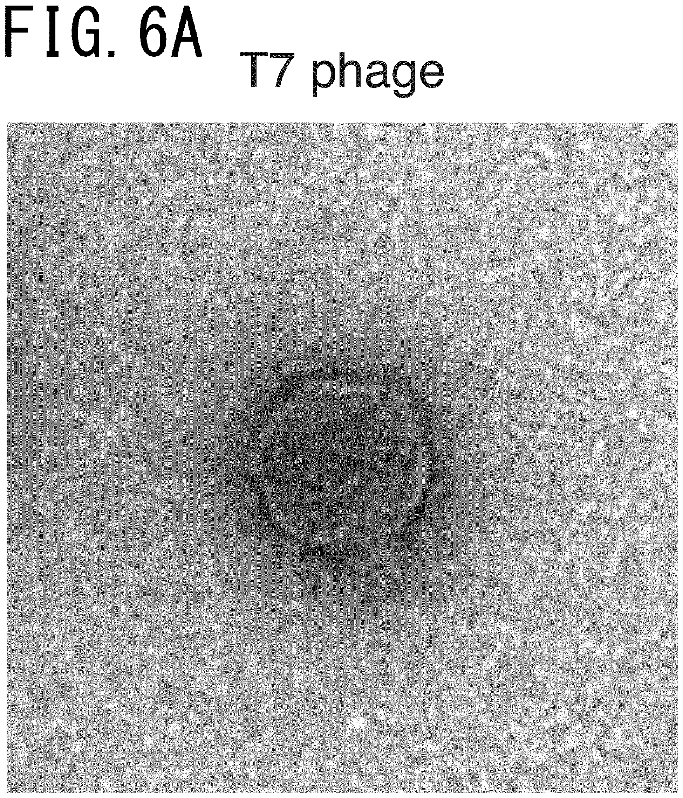

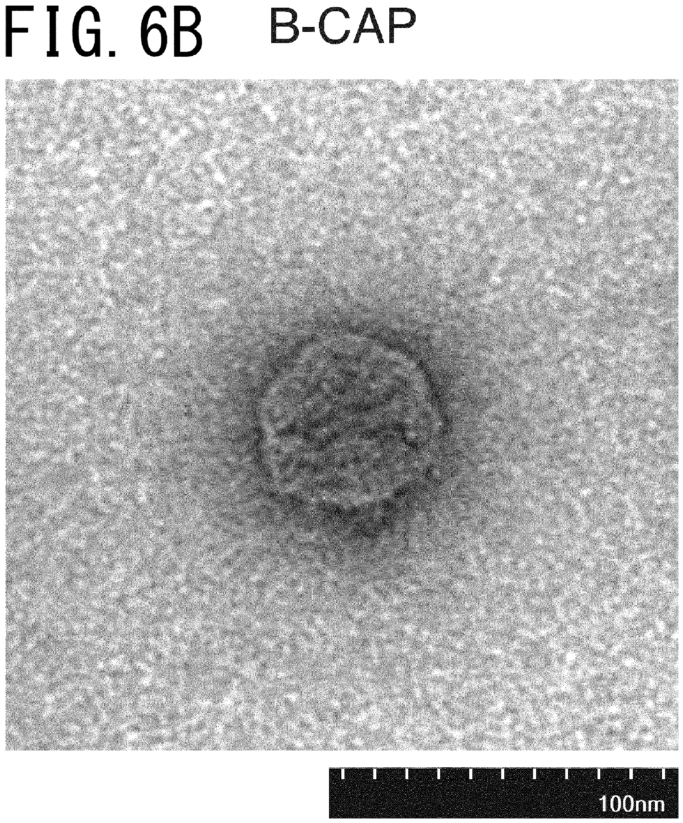

FIG. 6A and 6B shows the electron microscope photographs taken. FIG. 6A is a photograph of T7 bacteriophage, and FIG. 6B is a photograph of B-CAP.

As a result, it is confirmed that B-CAP is almost the same shape as T7 bacteriophage.

Then, the bactericidal activity of the constructed B-CAP is measured. Specifically, dilution series of T7 bacteriophage and B-CAP are prepared and dropped them onto E. coli MC1061 and MC1061 (pKLC172) cultured on a soft agar plate. After culturing at 37°C for 6 hours, a photograph of the plate is taken. All assays are performed three times.

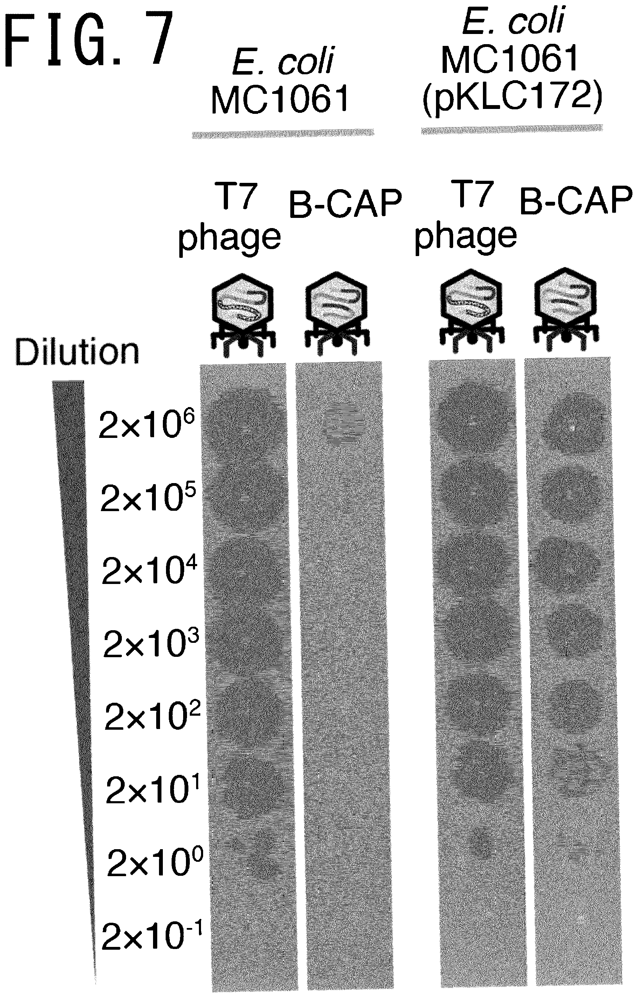

FIG. 7 shows the photographs taken. When B-CAP is infected into E. coli MC1061, bactericidal spots without bacteriophage plaques are observed only when 2×10⁵ or more is spotted on the bacterial lawn of MC1061. (bactericidal spots) are observed. B-CAP contains endolysin and toxic genes derived from T7 bacteriophage, so it is thought that bactericidal spots are formed differently from those of bacteriophages.

On the other hand, in the E. coli MC1061 (pKLC172) strain in which the genome region of the bacteriophage that B-CAP does not have is complemented with helper B-CAP (pKLC172), the growth of B-CAP is restored, and a bactericidal spot is formed even at a lower concentration of B-CAP. However, the plaque size caused by B-CAP is smaller than that of the complete T7 bacteriophage.

In the present example, B-CAP derived from Staphylococcus aureus is synthesized.

In this case, the genome of the Staphylococcus aureus Tan2 bacteriophage is divided into B-CAP DNA and helper B-CAP DNA. Specifically, the structural genes are deleted from the lysogenized Staphylococcus aureus Tan2 bacteriophage. Further, a helper B-CAP plasmid that expresses structural genes is constructed, and the structural gene-deficient Tan2 bacteriophage is transformed into the lysogenized Staphylococcus aureus RN4220. B-CAP is assembled by adding mitomycin C to this B-CAP-synthesizing bacteria and inducing the Tan2 bacteriophage.

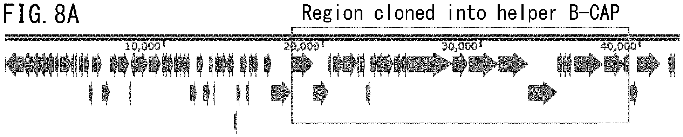

FIG. 8A to 8C shows an example of B-CAP construction from the Staphylococcus aureus Tan2 bacteriophage.

FIG. 8A shows the genetic structure of the Tan2 bacteriophage and the concept of the region cloned into the helper B-CAP. In this Tan2 bacteriophage, the 20140 bp region from the minor capsid to the tail fiber, which are the structural genes, is cloned and inserted into the pLC1 plasmid to construct the helper B-CAP plasmid for Tan2 bacteriophage.

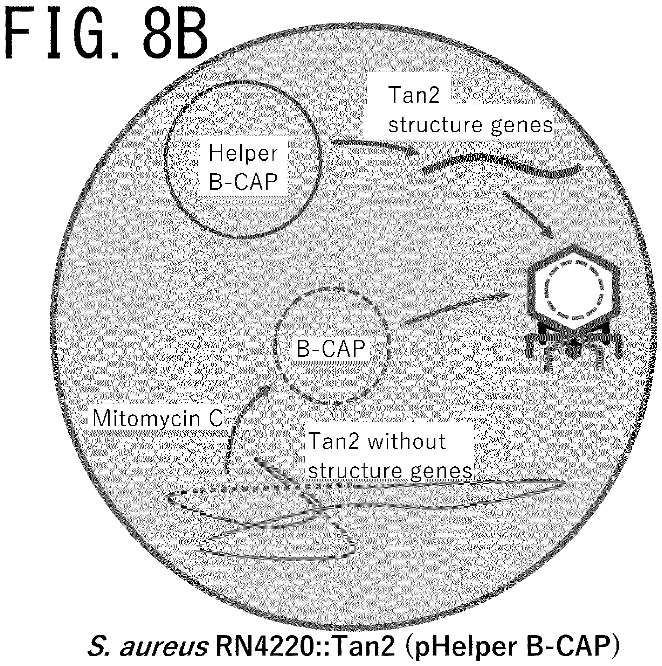

FIG. 8B shows an example of the construction of B-CAP by using this Helper B-CAP plasmid. Here, firstly, we generate a strain of Staphylococcus aureus RN4220 in which the Tan2 bacteriophage has been lysogenized. Then, we prepare a strain of "Tan2 without structural genes" in which the structural gene of the bacteriophage has been deleted from the bacteria. Then, the Helper B-CAP plasmid is transformed into RN4220 carrying the Tan2 without structural gene bacteriophage, and thus the construction of a Staphylococcus aureus strain that synthesizes Tan2-based B-CAP (We named this strain as "S. aureus RN4220: :Tan2 (pHelper B-CAP)"). B-CAP is assembled in this B-CAP-synthesizing strain by adding mitomycin C to induce the Tan2 bacteriophage.

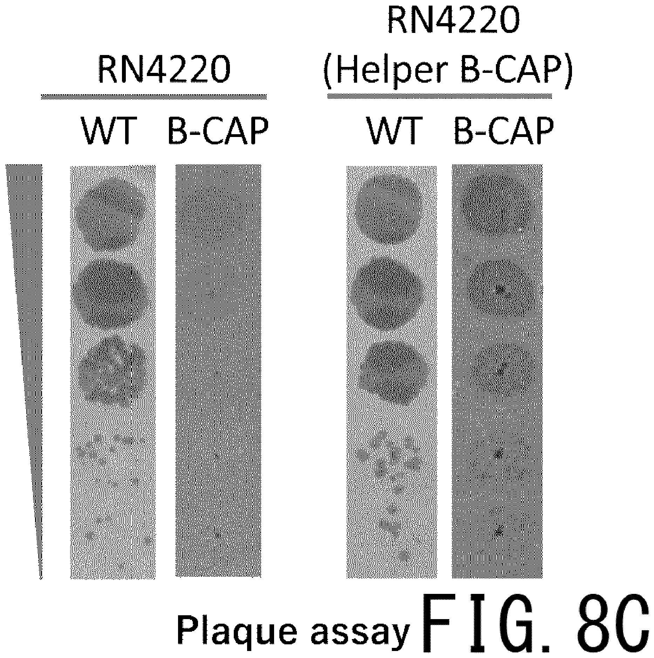

FIG. 8C shows: serial 10-fold dilutions of the prepared wild-type Tan2 bacteriophage and B-CAP are spotted onto agar plate containing RN4220 or RN4220 carrying the helper B-CAP plasmid (hereinafter referred to as "RN4220 (Helper B-CAP)"), and after overnight incubation, the formation of lytic plaques is observed. Since lytic plaques are observed only with RN4220 (Helper B-CAP), it is shown that the construction of B-CAP is successful.

### (Loading long DNA into B-CAP)

B-CAP can inject DNA into target bacteria by using the bacteriophage infection system. Therefore, the inventors of the present invention investigate the extent to which DNA of what length can be loaded onto B-CAP. Specifically, B-CAP genome has been divided into three fragments by PCR, and a DNA sequence derived from Staphylococcus aureus is inserted it.

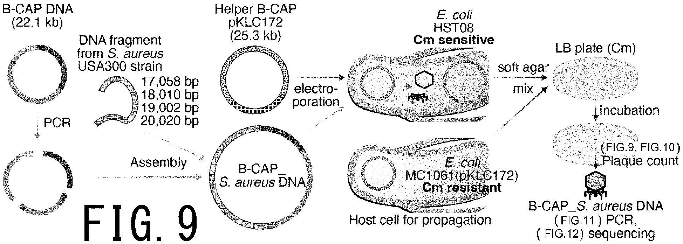

FIG. 9 shows the test for loading long DNA into B-CAP. Here, long DNA fragments derived from the Staphylococcus aureus chromosome are inserted into B-CAP to obtain B-CAP (S. aureus DNA). Specifically, DNA fragments of approximately 17, 18, 19, and 20 kb derived from Staphylococcus aureus are prepared and assembled into B-CAP DNA so that they would be inserted into the downstream region of gp19, which is the late gene of T7 bacteriophage. In the present experiment, B-CAP DNA is amplified into three fragments by using PCR, they are combined with DNA fragments from Staphylococcus aureus of 17 kb, 18 kb, 19 kb, and 20 kb, and then they are circularized. The circular DNA of B-CAP having the assembled Staphylococcus aureus sequence is electroporated into E. coli HST08 together with the helper B-CAP plasmid pKLC172. Afterwards, in order to detect the synthesized B-CAP (S. aureus DNA) as a plaque, the sample after electroporation is mixed with E. coli MC1061 (pKLC172) and soft agar and poured onto an LB plate containing chloramphenicol (Cm). The reason for adding chloramphenicol is to prevent the growth of the wild-type E. coli HST08 and to allow only the B-CAP helper E. coli MC1061 (pKLC172) to grow. After incubation, the plaques are observed.

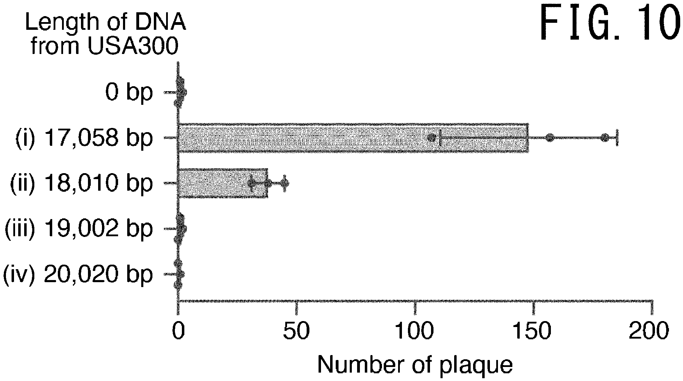

FIG. 10 shows the number of plaques observed by introducing long DNA of each length. Each bar shows the standard deviation and mean (n=3).

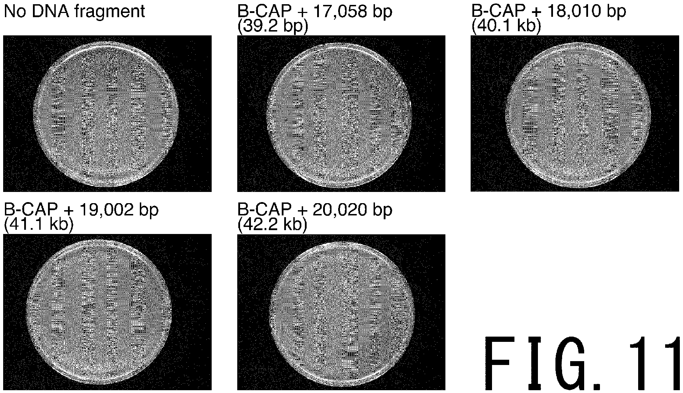

FIG. 11 shows a representative photograph of the B-CAP (S. aureus DNA) plaque.

When the number of plaques formed on the bacterial flora of MC1061 (pKLC172), which is a helper E. coli for B-CAP, is counted, approximately 150 and 40 plaques are observed for B-CAP with 17-kb and 18-kb DNA insertions, respectively.

On the other hand, a few plaques are also confirmed in B-CAPs attempted to insert 19kb and 20kb DNA, but all of these plaques are wild-type B-CAPs with no DNA inserted.

Here, we confirmed that the 17kb and 18kb DNA sequences from Staphylococcus aureus had been inserted into B-CAP by using PCR and Sanger sequencing.

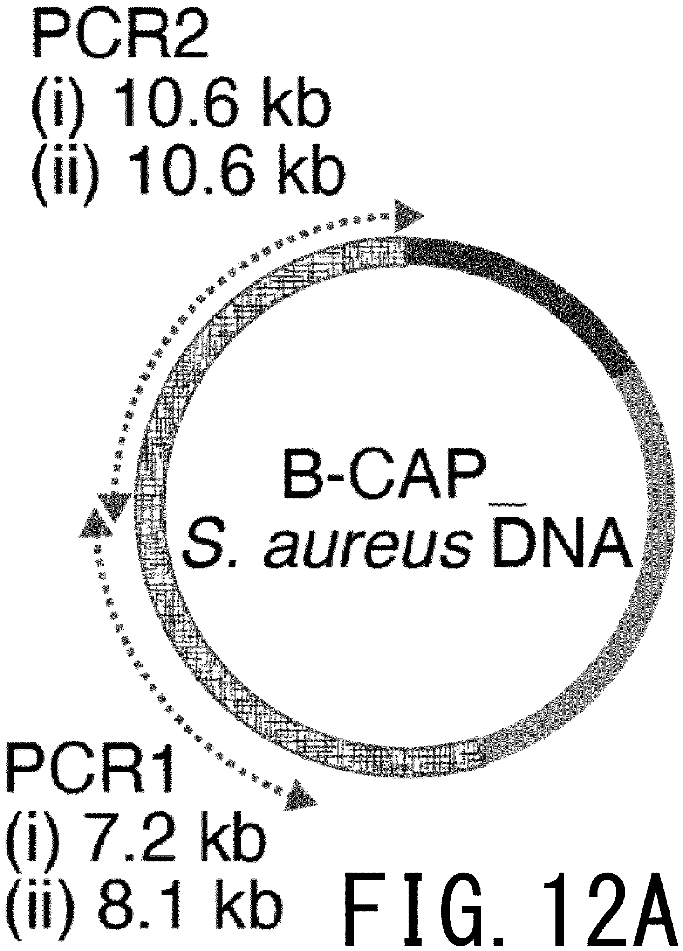

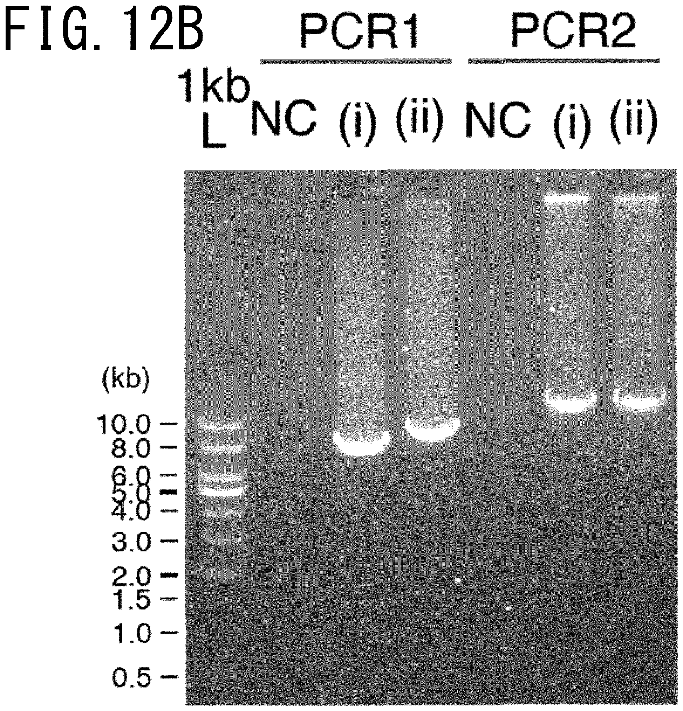

FIG. 12A and 12B shows the results of isolating and amplifying the obtained plaques and confirming the insertion of long DNA fragments by using the PCR method. FIG. 12A is a schematic diagram of each fragment. FIG. 12B shows the results of electrophoresis on a 0.5% agarose gel.

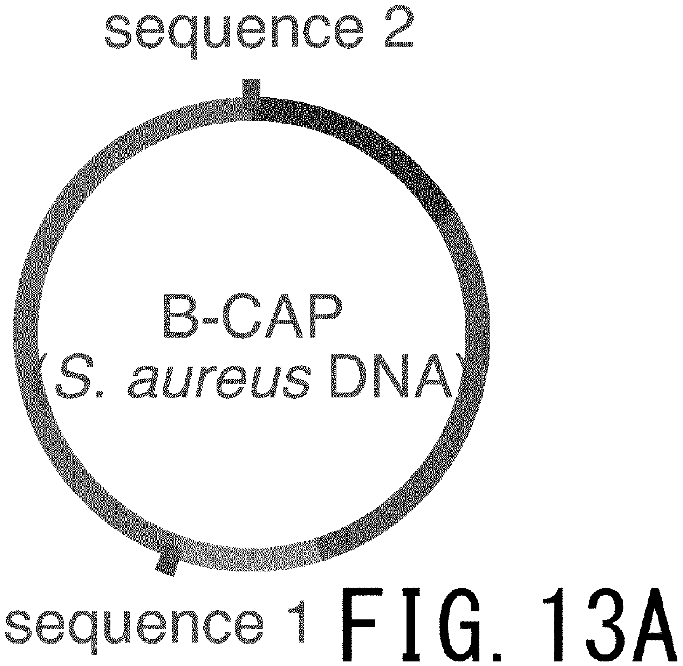

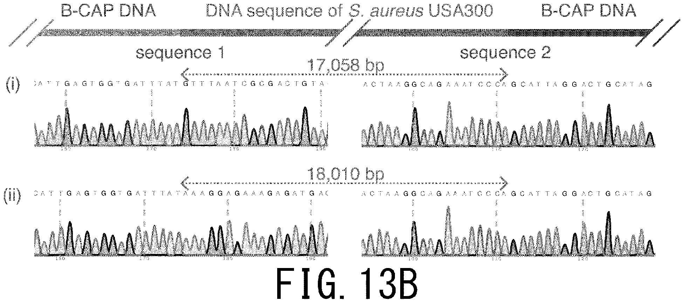

FIG. 13A and 13B shows the results of isolating and amplifying the plaques obtained in the same way and sequencing them by using the next-generation sequencer. FIG. 13A shows a schematic diagram of the positions where the B-CAP (S. aureus DNA) sequence is performed as sequence 1 and sequence 2. FIG. 13B shows part of the actual sequence.

From the above results, it is found that B-CAP can insert foreign DNA of at least 18 kb in length.

### (Construction of a powerful bactericidal B-CAP (NM-AB capsid) by loading a bactericidal gene)

Then, we attempted to develop an antibacterial agent by using B-CAP. As shown in the above FIG. 7, B-CAP contains a lysozyme enzyme and genes that alter the host's metabolism, thus, although it cannot proliferate, it is possible to kill infected bacteria.

Unlike ordinary bacteriophages, B-CAP does not multiply, so there is no risk of biological hazards such as unexpected infection or gene transmission. However, because it cannot multiply at the site of infection as like bacteriophages, it is expected to have less antibacterial power than ordinary bacteriophages. The inventors therefore considered adding antibacterial power to B-CAP.

In the present example, as a factor secreted outside the bacteria, colicin E1 (hereinafter referred to as "ColE1"), which is a bacteriocin that is bactericidal to E. coli and is encoded in the E. coli plasmid as disclosed in non-patent document 2 is used. ColE1 is encoded together with an immunity protein so that the bacteria itself that carries ColE1 is not killed, and it has a release protein to secrete bacteriocin outside the bacterium.

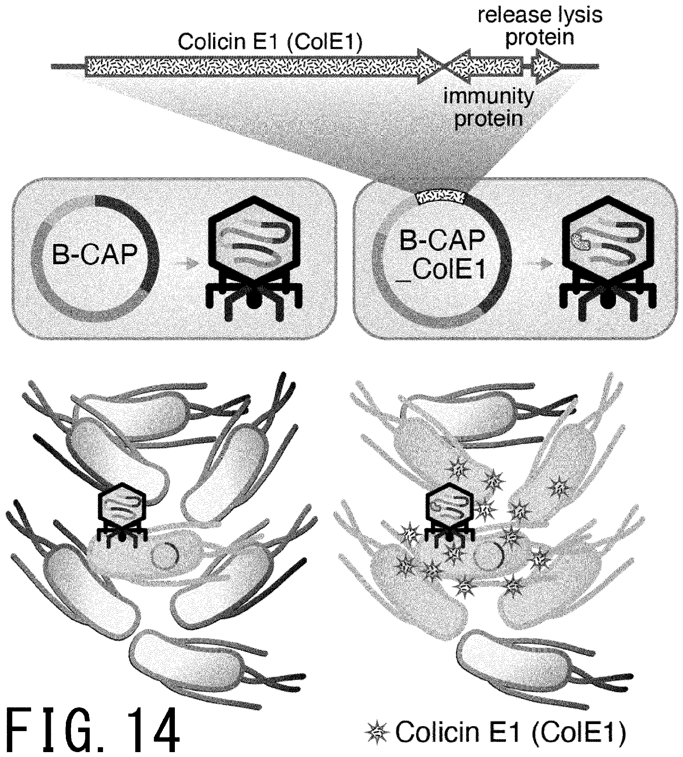

As refer to FIG. 14, the construction of the bacteriocin B-CAP (NM-AB capsid) in the present example is described.

In this example, the ColE1 gene region, which is a set of colicin, immunity protein, and release protein, is loaded onto B-CAP. The gene cassette, which is configured with the ColE1 gene, the immunity gene, and the release lysin protein gene, is loaded onto B-CAP, and the resulting NM-AB capsid structure is obtained and named B-CAP _ColE1. When B-CAP_ColE1 infects target bacteria and injects DNA into the cell, it produces the bactericidal ColE1 and secretes it into the surrounding area.

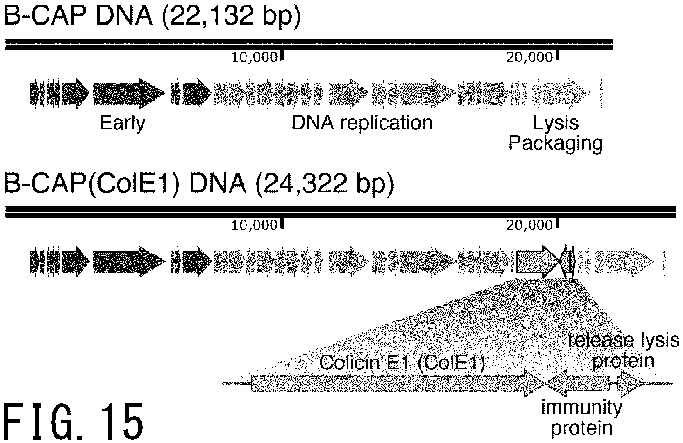

FIG. 15 shows the structure of the B-CAP (ColE1) sequence of the B-CAP_ColE1. Here, the entire DNA of the T7 bacteriophage and B-CAP is extracted, the sequence is read by sequencing by the next-generation sequencer, and the sequence is annotated by using RAST ver. 2.0. The genome length of B-CAP_ColE1 is 24322 bp. This confirmed that ColE1, immunity protein, and release protein are all inserted.

In the bacterial host into which B-CAP_ColE1 is injected, B-CAP_ColE1 DNA is transcribed. Then, ColE1, immunity protein, and release protein are produced, and the host bacteria secrete ColE1 outside the cell with the help of the release protein, while being protected by the immunity protein. It is predicted that the secreted ColE1 would be able to kill a wide range of bacteria in the area surrounding the host bacteria.

On the other hand, bacteria into which the DNA of B-CAP_ColE1 is injected are killed by toxic genes such as endolysin derived from B-CAP.

In this way, it is believed that it is possible to develop an antibacterial agent that is non-proliferative yet capable of killing a wide range of bacteria, thus, the bactericidal activity of B-CAP_ColE1 is investigated.

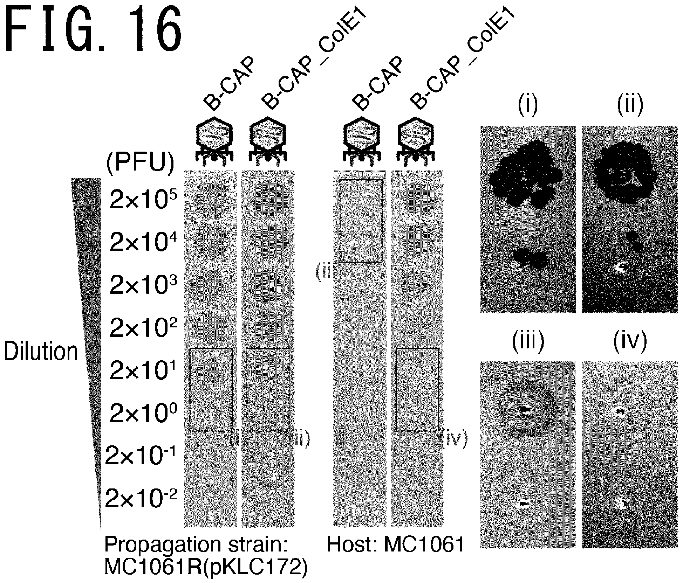

FIG. 16 shows the results of the investigation of the bactericidal power. The photograph shows the results of cultivating E. coli MC1061 and MC1061R (pKLC172) by planting them on a soft agar plate and then spotting solutions containing B-CAP and B-CAP_ColE1 on the plate, respectively. The "Propagation strain" is MC1061R (pKLC172), which is a strain that has introduced pKLC172 into the ColE1-resistant MC1061R strain, is a bacterium used to acquire B-CAP_COLE1, and is a comparative example. "Host: MC106" is bacteria used to actually evaluate for bactericidal power. These tests are repeated three times.

As a result, although B-CAP is non-proliferative, the bactericidal effect does not be confirmed very much. Alternatively, B-CAP_ColE1 shows an extraordinarily strong population bactericidal effect.

Specifically, when B-CAP is added to E. coli MC1061, a bactericidal spot is observed at 2×10⁵ PFU, but at lower concentrations, a bactericidal spot is almost never observed. On the other hand, with B-CAP_ColE1, a bactericidal spot is observed even at 2×10¹ PFU. Further, B-CAP_ColE1 formed small non-proliferative bactericidal spots. This is probably because the bacteria around the bacteria injected with B-CAP_ColE1 DNA are killed by ColE1.

Thus, the bactericidal power of B-CAP_ColE1 on the plate can be confirmed.

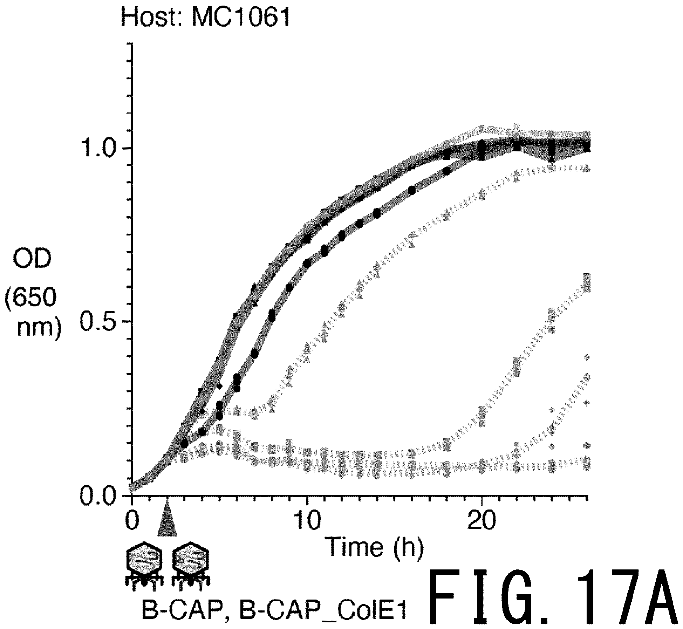

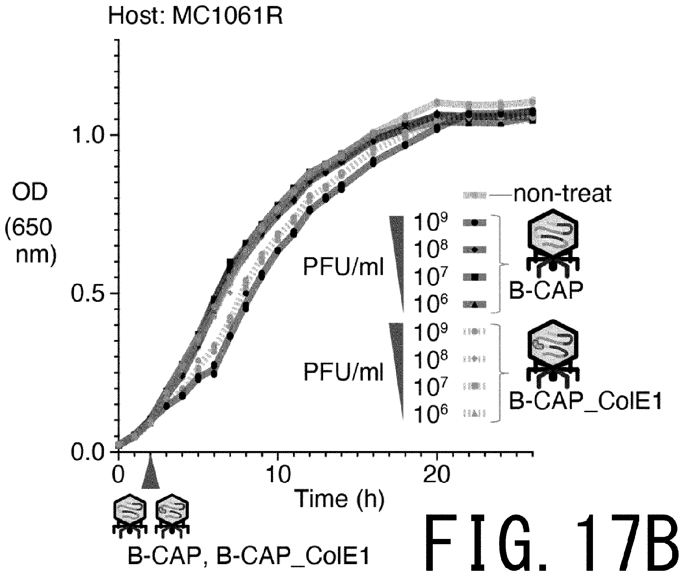

As refer to FIG. 17A and 17B, the results of an investigation into the bactericidal activity of B-CAP_ColE1 in liquid culture are described. The MC1061 strain and the T7-resistant MC1061R strain are liquidly cultured, and after 2 hours B-CAP and B-CAP_ColE1 are added at approximately MOI=1, 0.1, 0.01, and 0.001, and the growth of the bacteria is examined. Specifically, each bacterium in the growth phase is diluted and cultured, and after 2 hours, B-CAP or B-CAP_ColE1 is added so that the MOI is approximately 1, 0.1, 0.01, or 0.001.

Each series in FIG. 17A and 17B shows the average of the results of four independent experiments. FIG. 17A shows the results for MC1061, and FIG. 17B shows the results for MC1061R. When B-CAP is added to the MC1061 strain of E. coli at MOI=1, the growth curve becomes slightly gentler, but when it is added at MOI=0.1, 0.01, and 0.001, there is no change in the growth curve. The reason the growth curve becomes gentler when B-CAP is added at an MOI of 1 is presumed to be because MC1061 is killed by endolysin, or the like, encoded in the B-CAP DNA. On the other hand, B-CAP_ColE1 suppressed the growth of MC1061 with a much smaller amount than B-CAP.

The bactericidal effect of B-CAP_ColE1 at an MOI=0.001 is stronger than that of B-CAP at an MOI=1, and it is also found that B-CAP_ColE1 exhibits stronger antibacterial activity as the MOI is increased. The fact that the OD₆₀₀ decreased from 3 hours after the addition of B-CAP_ColE1 suggests that B-CAP_ColE1 acts bactericidally rather than bacteriostatically. Further, since almost no bactericidal activity is observed in the MC1061R strain, which is a ColE1-resistant E. coli, it is shown that the bactericidal effect of B-CAP_ColE1 on the MC1061 strain is due to ColE1.

### (Bactericidal effect of B-CAP on drug-resistant E. coli)

Drug resistance in Enterobacteriaceae bacteria, including E. coli, is a fundamental problem on a global level. In fact, Carbapenem-resistant Enterobacteriaceae (CRE) and ESBL-producing E. coli are classified as CRITICAL PRIORITY by the WHO; and CRE is classified as an Urgent Threat and ESBL-producing E. coli is classified as a Serious Threat by CDC, respectively.

We therefore investigate whether B-CAP_ColE1 is effective against clinical isolates of these drug-resistant E. coli.

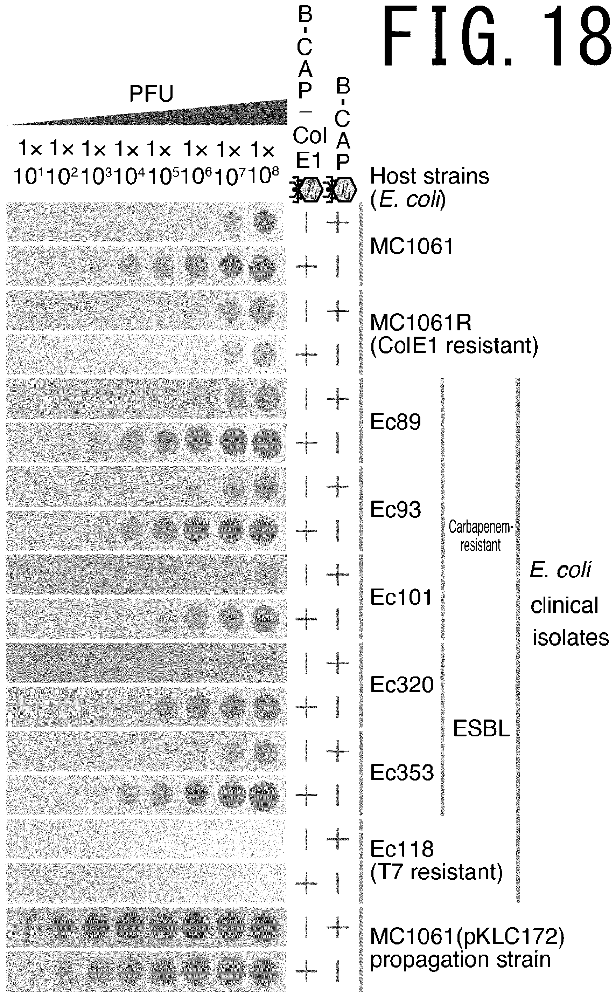

As refer to FIG. 18, the bactericidal effect of B-CAP_ColE1 on the plate is described. E. coli MC1061, ColE1-resistant MC1061R, clinical isolates of carbapenem-resistant E. coli, ESBL-producing E. coli, clinical isolates of T7-resistant E. coli, and MC1061 (pKLC172), which is a propagation strain, are inoculated onto soft agar plates. Dilution series of B-CAP and B-CAP (ColE1) are prepared, spotted onto the plates, and incubated at 37°C. Photographs are taken after incubation.

As a result, B-CAP_ColE1 effectively killed the clinical isolates of carbapenem-resistant E. coli, E89, E93, and E101. This bactericidal activity is almost the same as that against the laboratory strain, MC1061. Further, bactericidal spot with B-CAP is also observed, but the spot is thin, and we judge that the bactericidal activity is about 1/1000 that of B-CAP _ColE1. B-CAP_ColE1 also shows strong bactericidal activity against Ec320 and Ec353, which are multi-drug resistant bacteria, ESBL E. coli.

From these results, it is found that B-CAP_ColE1 has bactericidal activity against drug-resistant E. coli, which is a severe problem in clinical practice.

### (Antimicrobial therapeutic effect of bactericidal B-CAP on mice with bacterial infection)

Then, in order to investigate the antimicrobial therapeutic effect of B-CAP (ColE1), an antimicrobial therapeutic test for bacterial infection by using a mouse bacterial infection model is performed.

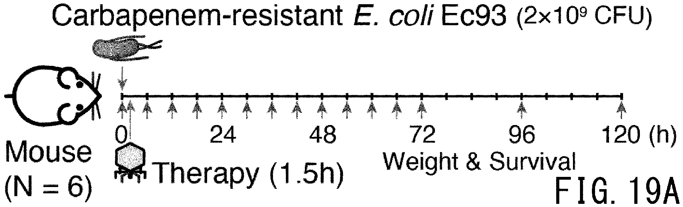

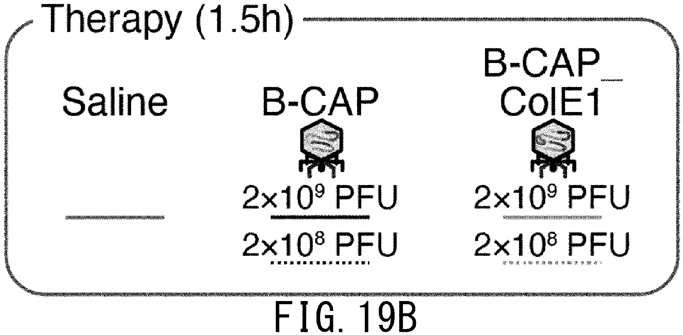

FIG. 19A and 19B explains this antimicrobial therapeutic test. FIG. 19A is a time series diagram, and FIG. 19B shows the dosage. In this study, 10 CFUs of a carbapenem-resistant E. coli strain (Ec93), which is a clinical isolate, are inoculated into the abdominal cavity of mice, and 1.5 hours later, B-CAP and B-CAP_ColE1 are administered directly into the abdominal cavity at an MOI=1 or 0.1, and the survival rate of the mice is examined.

In addition, a group is administered phosphate-buffered saline (PBS) as a negative control (shown as "Saline" in the figure), and a group is administered a proliferative T7 bacteriophage as a positive control.

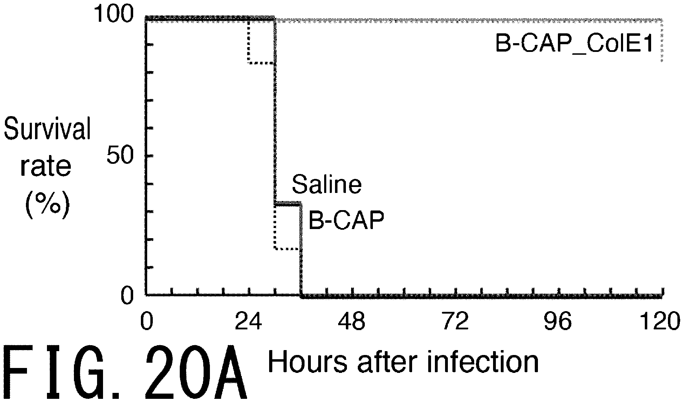

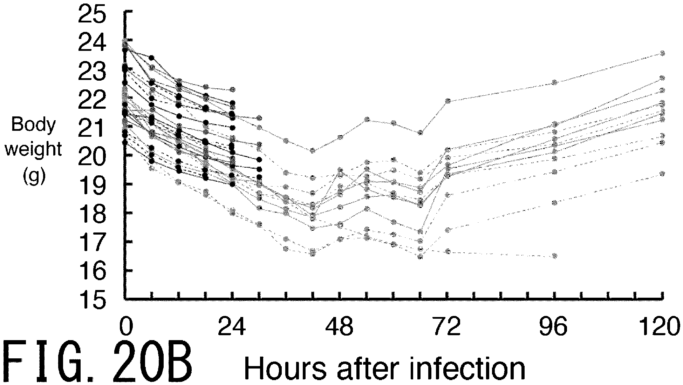

In FIG. 20A and 20B, as the antibacterial therapeutic effect of B-CAP_ColE1, the survival curve and weight change up to 120 hours after administration is shown. FIG. 20A is the survival curve and FIG. 20B is the weight change graph. Survival and weight are confirmed every 6 hours up to 72 hours after infection, and then at 96 hours and 120 hours after infection.

There is no difference between the B-CAP and PBS (saline) groups, but there is a significant difference between the B-CAP_ColE1 and PBS (saline) groups. Here, the p-value is calculated by using the log-rank test (Mantel-Cox test). The results are expressed as the sum of three independent experiments performed with six mice per group. The result is p=0.0006.

All mice in the B-CAP and negative control PBS (saline) groups died between 18 and 30 hours after infection. On the other hand, in the B-CAP_ColE1 treatment group, one mouse died at 120 hours after infection at an MOI=0.1, but all the other mice survived.

Further, the results of the weight measurements over time showed that the treatment success group peaked at around 42 hours and then gradually recovered. The group that is administered the proliferative T7 bacteriophage, which is used as a positive control, also recovered in all cases.

These results suggest that B-CAP_ColE1 can be used for the antimicrobial treatment of drug-resistant E. coli, even though it is non-proliferative, and that it is effective even in small doses.

### (Summary)

In the present example, B-CAP (ColE1) is constructed by simultaneously loading a bacteriocin, a secretory gene, and an immune gene into B-CAP, and it is an antibacterial preparation that can synthesize a large amount of bacteriocin inside the cells of B-CAP-infected bacteria and secrete it outside the cells. Furthermore, B-CAP_ColE1 showed strong therapeutic efficacy against a mouse E. coli peritoneal infection model even at an MOI=0.1. B-CAP is a non-proliferative product that ensures safety, and this product, which achieves therapeutic efficacy at an MOI=0.1, is thought to be a highly effective move in bacteriophage therapy, which has issues with safety and treatment accuracy.

### (Industrial applicability)

According to the present disclosure, bacteria-targeting capsid particles (B-CAPs) can be provided as a treatment composition, a disinfectant, a food, and the like, and are industrially applicable.

## Claims

1. A bacteria-targeting capsid particle comprising:
a capsid protein of a bacteriophage; and
a bacteria-targeting capsid particle element including a nucleic acid injection region, a replication region necessary for replication of nucleic acid, and a packaging region from genome of the bacteriophage; and wherein the bacteria-targeting capsid particle is non-proliferative.

2. The bacteria-targeting capsid particles according to claim 1, wherein
the bacteria-targeting capsid particle element includes an exogenous gene.

3. The bacteria-targeting capsid particles according to claim 2, wherein
the exogenous gene includes one or any combination of a bactericidal gene, a biofilm-degrading gene, an antigen-presenting gene, or a gene for introduction.

4. The bacteria-targeting capsid particles according to claim 3, wherein
the bactericidal gene produces a secretory bactericidal product that sterilizes surrounding bacteria.

5. The bacteria-targeting capsid particles according to claim 4, wherein
the exogenous gene includes a resistance factor that suppresses the bactericidal effect on the target bacteria.

6. A treatment composition comprising:
the bacteria-targeting capsid particle according to any one of claims 1 to 5.

7. A disinfectant comprising:
the bacteria-targeting capsid particle according to any one of claims 1 to 5.

8. A food comprising:
the bacteria-targeting capsid particle according to any one of claims 1 to 5.

9. A bacteria elimination method comprising the step of:
sterilizing target bacteria by the bacteria-targeting capsid particle according to any one of claims 1 to 5.

10. The bacteria elimination method according to claim 9, wherein
the target bacteria are present in bacterial flora of a human, an animal, and/or an environment.

11. The bacteria elimination method according to claim 9, wherein
the target bacteria are present in food.

12. A bactericidal method comprising the step of:
sterilizing target bacteria with the bacteria-targeting capsid particle according to any one of claims 1 to 5.

13. A corrosion prevention method comprising the steps of:
sterilizing target bacteria with the bacteria-targeting capsid particle according to any one of claims 1 to 5; and
preventing corrosion of an article.

14. An animal treatment method comprising the step of:
treating an animal with the bacteria-targeting capsid particle according to any one of claims 1 to 5.

15. A gene introduction method comprising the step of:
introducing the exogenous gene included in the bacteria-targeting capsid particle according to any one of claims 2 to 5 into the target bacteria.

16. A bacteria function addition method comprising the steps of:
introducing the exogenous gene included in the bacteria-targeting capsid particle according to any one of claims 2 to 5 into the target bacteria; and
adding a function to the target bacteria.

17. A method for producing bacteria-targeting capsid particle comprising the steps of:
preparing a capsid protein of a bacteriophage using a capsid nucleic acid element, which synthesizes a capsid without including a packaging region and is divided from genome of the bacteriophage;
packaging a bacteria-targeting capsid particle element, which includes a nucleic acid injection region, a replication region necessary for nucleic acid replication, and a packaging region and is divided from a part of the genome of the bacteriophage other than the capsid nucleic acid element, into the capsid protein.

18. The method for producing the bacteria-targeting capsid particles according to claim 17, further comprising the steps of:
introducing the capsid nucleic acid element and the bacteria-targeting capsid particle element into a preparation bacteria for preparation; and
producing the bacteria-targeting capsid particles in the preparation bacteria.

19. The method for producing the bacteria-targeting capsid particles according to claim 18, wherein
introducing the capsid nucleic acid element by a chromosome or an artificial chromosome for the preparation bacteria.

20. The method for producing the bacteria-targeting capsid particles according to claim 19, wherein
introducing the bacteria-targeting capsid particle element by the chromosome or a plasmid.

21. A method for producing nucleic acid for bacteria-targeting capsid particle comprising the steps of:
preparing a capsid nucleic acid element that synthesizes a capsid without including a packaging region by dividing a bacteriophage genome;
preparing a bacteria-targeting capsid particle element including a nucleic acid injection region, a replication region necessary for replication of nucleic acid, and the packaging region by dividing a part of the bacteriophage genome other than the capsid nucleic acid element; and
constructing a nucleic acid for the bacteria-targeting capsid particle that is non-proliferative.
